# EUROPÄISCHE PATENTSCHRIFT

(11) **EP 3 601 203 B1**
(45) Veröffentlichungstag und Bekanntmachung des Hinweises auf die Patenterteilung: **07.04.2021**
(21) Anmeldenummer: 18708380.3
(22) Anmeldetag: 23.02.2018
(51) Int. Cl.: C07C 17/354, C07C 23/10, C07C 69/75

(54) **VERFAHREN ZUR HERSTELLUNG FLUORIERTER CYCLISCHER ALIPHATISCHER VERBINDUNGEN**
PROCESS FOR SYNTHESIZING FLUORINATED CYCLIC ALIPHATIC COMPOUNDS
PROCÉDÉ DE FABRICATION DE COMPOSÉS ALIPHATIQUES CYCLIQUES FLUORÉS

(30) Priorität: 27.03.2017 DE 102017106467
(43) Veröffentlichungstag der Anmeldung: 05.02.2020
(73) Patentinhaber: Westfälische Wilhelms-Universität Münster, 48149 Münster (DE)
(72) Erfinder: WIESENFELDT, Mario Patrick, 67125 Dannstadt (DE); GLORIUS, Frank, 48149 Münster (DE); NAIROUKH, Zackaria, 48149 Münster (DE)
(74) Vertreter: Michalski Hüttermann & Partner Patentanwälte mbB
(86) Internationale Anmeldenummer: PCT/EP2018/054554
(87) Internationale Veröffentlichungsnummer: WO 2018/177661

(56) Entgegenhaltungen:
- DE OLIVEIRA P R ET AL: "1,3-Diaxial steric effects and intramolecular hydrogen bonding in the conformational equilibria of new cis-1,3-disubstituted cyclohexanes using low temperature NMR spectra and theoretical calculations", SPECTROCHIMICA ACTA. PART A: MOLECULAR AND BIOMOLECULAR SPECTROSCOPY, ELSEVIER, AMSTERDAM, NL, Bd. 62, Nr. 1-3, 1. November 2005 (2005-11-01), Seiten 30-37, XP027703475, ISSN: 1386-1425 [gefunden am 2005-11-01]
- HONG YANG ET AL: "Hydrodefluorination of Fluorobenzene and 1,2-Difluorobenzene under Mild Conditions over Rhodium Pyridylphosphine and Bipyridyl Complexes Tethered on a Silica-Supported Palladium Catalyst", ORGANOMETALLICS, Bd. 18, Nr. 12, 1. Juni 1999 (1999-06-01), Seiten 2285-2287, XP055463690, US ISSN: 0276-7333, DOI: 10.1021/om990151e
- FACHE F ET AL: "A Catalytic Stereo- and Chemo-Selective Method for the Reduction of Substituted Aromatics", TETRAHEDRON LETTERS, ELSEVIER, AMSTERDAM, NL, Bd. 36, Nr. 6, 6. Februar 1995 (1995-02-06), Seiten 885-888, XP004028696, ISSN: 0040-4039, DOI: 10.1016/0040-4039(94)02386-P
- ALLEN D P ET AL: "Rhodium N-heterocyclic carbene complexes: Synthesis, structure, NMR studies and catalytic activity", JOURNAL OF ORGANOMETALLIC CHEMISTRY, ELSEVIER-SEQUOIA S.A. LAUSANNE, CH, Bd. 690, Nr. 24-25, 1. Dezember 2005 (2005-12-01), Seiten 5736-5746, XP027709172, ISSN: 0022-328X [gefunden am 2005-12-01]
- WOLFGANG A HERRMANN ET AL: "Synthesis and Characterization of N-Heterocyclic Carbene Substituted Phosphine and Phosphite Rhodium Complexes and their Catalytic Properties in Hydrogenation Reactions", ADVANCED SYNTHESIS & CATAL, WILEY-VCH VERLAG GMBH, DE, Bd. 349, Nr. 10, 2. Juli 2007 (2007-07-02) , Seiten 1677-1691, XP008111210, ISSN: 1615-4150, DOI: 10.1002/ADSC.200700079
- YU WEI ET AL: "Highly Selective Hydrogenation of Aromatic Ketones and Phenols Enabled by Cyclic (Amino)(alkyl)carbene Rhodium Complexes", JOURNAL OF THE AMERICAN CHEMICAL SOCIETY, Bd. 137, Nr. 29, 29. Juli 2015 (2015-07-29), Seiten 9250-9253, XP055463658, ISSN: 0002-7863, DOI: 10.1021/jacs.5b05868

## Beschreibung

Die vorliegende Erfindung bezieht sich auf das Gebiet fluorierter aliphatischer Cycloverbindungen. Derartige Verbindungen spielen eine große Rolle im Pharma- oder Agrochemiebereich aufgrund der Polarität der C-F-Bindung bei gleichzeitiger sterischer Ähnlichkeit zu C-H-Bindungen und anderen Effekten. Weiterhin sind additive Dipoleffekte bei mehreren gleich ausgerichteten C-F-Bindungen interessant für materialchemische Anwendungen.

Trotz der Wichtigkeit dieser Verbindungen sind effektive Methoden zur Herstellung nicht immer gegeben, so dass ein ständiger Bedarf nach Syntheseverfahren von fluorierten aliphatischen Cycloverbindungen besteht.

Es ist somit eine Aufgabe, neuartige Verfahren zur Herstellung derartiger Verbindungen bereitszustellen. Diese Aufgabe wird durch ein Verfahren gemäß Anspruch 1 gelöst. Demgemäß wird ein Verfahren zur Herstellung fluorierter cyclischer aliphatischer Verbindungen bereitgestellt, umfassend den Schritt der Hydrierung einer aromatischen fluorhaltigen Vorläufersubstanz mit einem Katalysator, umfassend mindestens ein Rhodiumatom sowie mindestens einen N-heterocyclischen Carbenliganden, in Gegenwart eines Reduktionsmittels.

Der Stand der Technik kennt verwandte Verfahren, allerdings mit zum vorliegenden Verfahren unterschiedlichen Katalysatoren wie z.B. Rhodiumoxid-, Rhodium-Dipyridyl- und Ruthenium (III)-Katalysatoren, welche die gewünschten fluorierten aliphatischen Cycloverbindungen in niedrigen bis mässigen Ausbeuten liefern, siehe De Oliveira, Spectrochimica Acta, Part A, Bd. 62, Nr. 1-3 (1.11.2005), Seiten 30-37, Hong Yang, Organometallics, Bd. 18, Nr. 12 (1.6.1999), Seiten 2285-2287 und Fache, Tetrahedron Letters, Bd. 36, Nr. 6 (6.2.1995), Seiten 885-888.

Überraschenderweise hat sich herausgestellt, dass durch das erfindungsgemässe Verfahren, oftmals in hervorragender Ausbeute, die entsprechenden fluorhaltigen aliphatischen Cycloverbindungen erhältlich sind und ein F-H-Austausch, wie er bei vielen analogen Hydrierungen zu erwarten gewesen wäre, entweder gar nicht oder nur soweit erfolgt, dass trotzdem das fluorhaltige Produkt in akzeptabler Ausbeute gewonnen werden kann.

Überraschenderweise werden dabei bei vielen Anwendungen eine ganze Reihe von funktionalen Gruppen toleriert wie z.B. Estergruppen oder geschützte Amin/Hydroxygruppen. Weiterhin überraschend hat sich herausgestellt, dass für den Fall, dass die Ausgangssubstanz zwei oder mehr Fluoratome enthält, bei vielen Anwendungen bevorzugt das *cis*-Produkt entsteht, was den Zugang zu sterisch definierten Produkten ermöglicht.

Unter dem Term "aromatische fluorhaltige Substanz" werden insbesondere fluorierte substituierte Benzolderivate, fluorierte carbo-oder heterozyklische 5-oder 6-Ringe mit π-Elektronensextett sowie fluorierte annelierte aromatische Multizyklen verstanden.

Es sei darauf hingewiesen, dass unter dem Term "aromatische fluorhaltige Substanz" insbesondere verstanden wird, dass sich zumindest ein Fluor als Substituent an einem aromatischen Ring befindet.

Besonders bevorzugt sind aromatische fluorhaltige Vorläufersubstanzen, welche keine heteroaromatischen Ringe enthalten.

Alternativ bevorzugt sind aromatische fluorhaltige Vorläufersubstanzen, welchen einen oder mehrere benzanellierte heteroaromatische Ringe enthalten.

Unter dem Term "Hydrierung" wird insbesondere verstanden, dass in der Summe zwei Wasserstoffe an eine "Doppelbindung" der aromatischen Verbindung addiert werden. Die vorliegende Erfindung ist jedoch nicht auf Wasserstoff als konkretes Reduktionsmittel beschränkt, auch wenn dies (wie im folgenden beschrieben) eine bevorzugte Ausführungsform der Erfindung ist.

Unter dem Term "N-heterocyclischer Carbenligand" werden elektronenreiche, nukleophile Verbindungen von zweiwertigen Kohlenstoffspezies mit Elektronensextett verstanden, wie z. B. Pyrrolidinylidene, Pyrrolidene, Imidazolylidene, Imidazolidinylidene, Piperidinylene, Hexahydropyrimidinylene und Triazolylidene. Besonders bevorzugt sind dabei Pyrrolidinylidene, Pyrrolidene, Imidazolylidene und Imidazolidinylidene, weiter bevorzugt Pyrrolidinylidene.

Für den Fall, dass der N-heterocyclische Carbenligand eine Pyrrolidinyliden-Verbindung umfasst, ist es insbesondere bevorzugt, dass sich in α-Stellung sowohl zum Stickstoff wie zum Carben kein Wasserstoff befindet. Dies hat sich bei den meisten Anwendungen der vorliegenden Erfindung als besonders effektiv für die Hydrierung herausgestelt, da so die Aktivität des Katalysators nicht durch Enaminbildung etc. herabgesenkt wird.

Gemäß einer bevorzugten Ausführungsform wird der Katalysator in Substanz eingesetzt. Es kann jedoch gemäß alternativen Ausführungsformen auch vorteilhaft sein, den Katalysator in situ herzustellen. Somit umfasst gemäß einer bevorzugten Ausführungsform der Erfindung das erfindungsgemäße Verfahren zwei Schritte:
a) *in situ* Synthese des in Schritt b) eingesetzten Katalysators aus geeigneten Vorstufen
b) Durchführen der Hydrierung wie oben und im weiteren beschrieben

Als geeignete rhodiumhaltige Vorstufe kommt insbesondere das Cyclooctadienrhodiumchloriddimer ([Rh(COD)Cl]₂) in Frage.

Bevorzugt wird Schritt a) durchgeführt, indem ein geeignetes Salz des N-heterocyclischen Carbens mit einer starken Base, vorzugsweise ausgewählt aus NaOtBu, LDA, KOtBu, NaH, KHMDS, LiHMDS mit einer Rhodium entaltenden Vorstufe, beispielsweise [Rh(COD)Cl]₂, ggf. bei erhöhter Temperatur umgesetzt wird. Als Anionen können hierbei Halogenide, Pseudohalogenide wie sauerstoffhaltige Anionen, insbesondere Triflate und schwach koordinierende Anionen wie Tetrafluoroborat, Hexafluoroantimonat oder Hexafluorophosphat dienen. Bevorzugte Lösemittel sind hierbei Hexan, Diethylether oder Tetrahydrofuran.

Für den Fall, dass in Schritt b) ein anderes Lösemittel verwendet wird als in Schritt a) wird ggf. zwischen den beiden Schritten dann das Lösemittel entfernt.

Gemäß einer bevorzugten Ausführungsform der Erfindung umfasst der Katalysator mindestens einen N-heterocyclischen Carbenliganden der folgenden Struktur: 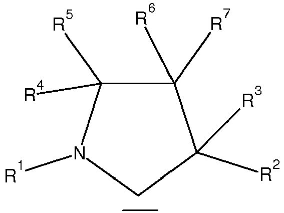 wobei
R¹ bis R⁵ unabhängig voneinander Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei können, wobei bei geeigneten Resten eine oder mehrere nicht-benachbarte CH₂-Gruppen unabhängig voneinander durch -O-, -S-, -NH-, -NR°-, -SiR°R°°-, -CO-, -COO-, -OCO-, -OCO-O-, -SO₂-, -S-CO-, -CO-S-, -CY¹=CY² oder -C=C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
R⁶ und R⁷ unabhängig voneinander Wasserstoff, Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei können, wobei bei geeigneten Resten eine oder mehrere nicht-benachbarte CH₂-Gruppen unabhängig voneinander durch -O-, -S-, -NH-, -NR°-, -SiR°R°°-, -CO-, -COO-, -OCO-, -OCO-O-, -SO₂-, -S-CO-, -CO-S-, -CY¹=CY² oder -C=C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
und R¹ bis R⁷ so substituiert sein können, dass sich wahlweise zwischen R² und R³, R⁴ und R³, R⁶ und R⁷, R¹ und R⁴/R⁵, R⁴/R⁵ und R⁶/R⁷ oder R²/R³ und R⁶/R⁷ ein Ring bildet.
allgemeine Gruppendefinition: Innerhalb der Beschreibung und den Ansprüchen werden allgemeine Gruppen, wie z.B: Alkyl, Alkoxy, Aryl etc. beansprucht und beschrieben. Wenn nicht anders beschrieben, werden bevorzugt die folgenden Gruppen innerhalb der allgemein beschriebenen Gruppen im Rahmen der vorliegenden Erfindung verwendet:
   alkyl: lineare und verzweigte Cl-C8-Alkyle,
   langkettige Alkyle: lineare und verzweigte C5-C20 Alkyle
   alkenyl: C2-C6-alkenyl,
   cycloalkyl: C3-C8-cycloalkyl,
   alkoxy: C1-C6-alkoxy,
   langkettig Alkoxy: lineare und verzweigte C5-C20 Alkoxy
   alkylene: ausgewählt aus der Gruppe enthaltend:
      methylene; 1,1-ethylene; 1,2-ethylene; 1,1-propylidene; 1,2-propylene; 1,3- propylene; 2,2-propylidene; butan-2-ol-1,4-diyl; propan-2-ol-1,3-diyl; 1, 4-butylene; cyclohexane-1,1-diyl; cyclohexan-1,2-diyl; cyclohexan-1,3-diyl; cyclohexan-1,4-diyl; cyclopentane-1,1-diyl; cyclopentan-1,2-diyl; und cyclopentan-1,3-diyl,
      aryl: ausgewählt aus Aromaten mit einem Molekulargewicht unter 300Da
      arylene: ausgewählt aus der Gruppe enthaltend: 1,2-phenylene; 1,3- phenylene; 1,4-phenylene; 1,2-naphtalenylene; 1,3-naphtalenylene; 1,4- naphtalenylene; 2,3-naphtalenylene; 1-hydroxy-2,3-phenylene; 1-hydroxy-2,4- phenylene; 1-hydroxy-2,5- phenylene; und 1-hydroxy-2,6-phenylene,
      heteroaryl: ausgewählt aus der Gruppe enthaltend: pyridinyl; pyrimidinyl; pyrazinyl; triazolyl; pyridazinyl; 1,3,5-triazinyl; chinoninyl; isochinoninyl; chinoxalinyl; imidazolyl; pyrazolyl; benzimidazolyl; thiazolyl; oxazolidinyl; pyrrolyl; thiophenyl; benzothiophenyl, furyl, benzofuryl, carbazolyl; indolyl; und isoindolyl, wobei das Heteroaryl mit der Verbindung über jedes Atom im Ring des ausgewählten Heteroaryls verbunden sein kann.
      heteroarylene: ausgewählt aus der Gruppe enthaltend: pyridindiyl; quinolindiyl; pyrazodiyl; pyrazoldiyl; triazolediyl; pyrazindiyl, thiophendiyl; und imidazolediyl, wobei das
      heteroarylene als Brücke in der Verbindung über ein beliebiges Atom im Ring des ausgewählten Heteroaryls fungiert, speziell bevorzugt sind: pyridin-2, 3-diyl; pyridin-2,4-diyl; pyridin-2,5-diyl; pyridin-2,6-diyl; pyridin-3,4- diyl; pyridin-3,5-diyl; quinolin-2,3-diyl; quinolin-2,4-diyl; quinolin-2, 8-diyl; isoquinolin-1,3-diyl; isoquinolin-1,4-diyl; pyrazol-1,3-diyl; pyrazol-3,5- diyl; triazole-3,5-diyl; triazole-1,3-diyl; pyrazin-2,5-diyl; und imidazole-2,4-diyl, thiophen-2,5-diyl, thiophen-3,5-diyl; ein -C1-C6-heterocycloalkyl, ausgewählt aus der Gruppe enthaltend: piperidinyl; piperidine; 1,4-piperazine, tetrahydrothiophene; tetrahydrofuran; 1,4,7-triazacyclononane; 1,4,8,11- tetraazacyclotetradecane; 1,4,7,10,13-pentaazacyclopentadecane; 1,4-diaza- 7-thia-cyclononane; 1,4- diaza-7-oxa-cyclononane; 1,4,7,10-tetraazacyclododecane; 1,4-dioxane; 1,4, 7-trithia-cyclononane; pyrrolidine; und tetrahydropyran, wobei das Heteroaryl mit dem C1-C6-Alkyl über jedes Atom im Ring des ausgewählten Heteroaryls verbunden sein kann.
      heterocycloalkylene: ausgewählt aus der Gruppe enthaltend: piperidin-1,2- ylene; piperidin-2,6-ylene; piperidin-4,4-ylidene; 1,4-piperazin-1,4-ylene; 1,4-piperazin-2,3-ylene; 1,4-piperazin-2,5-ylene; 1,4-piperazin-2,6-ylene; 1,4-piperazin- 1,2-ylene; 1,4-piperazin-1,3-ylene; 1,4-piperazin-1,4-ylene; tetrahydrothiophen-2,5-ylene; tetrahydrothiophen-3,4-ylene; tetrahydrothiophen-2,3-ylene; tetrahydrofuran-2,5-ylene; tetrahydrofuran- 3,4-ylene; tetrahydrofuran-2,3-ylene; pyrrolidin-2,5-ylene; pyrrolidin-3,4-ylene; pyrrolidin-2,3-ylene; pyrrolidin-1,2-ylene; pyrrolidin-1,3-ylene; pyrrolidin-2,2-ylidene; 1,4,7-triazacyclonon-1,4-ylene; 1,4,7- triazacyclonon-2,3-ylene; 1,4,7-triazacyclonon-2,9-ylene; 1,4,7-triazacyclonon-3,8-ylene; 1,4,7-triazacyclonon-2,2- ylidene; 1,4,8,11-tetraazacyclotetradec-1,4-ylene; 1,4,8,11- tetraazacyclotetradec-1,8-ylene; 1,4,8,11-tetraazacyclotetradec-2,3-ylene; 1,4,8,11-tetraazacyclotetradec-2,5-ylene; 1,4,8,11- tetraazacyclotetradec-1,2-ylene; 1,4,8,11-tetraazacyclotetradec-2,2-ylidene; 1,4,7,10-tetraazacyclododec-1,4-ylene; 1,4,7,10-tetraazacyclododec-1,7-ylene; 1,4,7,10-tetraazacyclododec-1,2- ylene; 1,4,7,10-tetraazacyclododec-2,3- ylene; 1,4,7,10-tetraazacyclododec-2,2-ylidene; 1,4,7,10,13 pentaazacyclopentadec-1,4-ylene; 1,4,7,10,13- pentaazacyclopentadec-1,7-ylene; 1,4,7,10,13-pentaazacyclopentadec-2,3- ylene; 1,4,7,10,13-pentaazacyclopentadec-1,2-ylene; 1,4,7,10,
      13-pentaazacyclopentadec-2,2-ylidene; 1,4-diaza-7-thia-cyclonon- 1,4-ylene; 1,4-diaza-7-thia-cyclonon-1,2-ylene; 1,4-diaza-7thia-cyclonon- 2,3-ylene; 1,4-diaza-7-thia-cyclonon-6,8-ylene; 1,4-diaza-7-thia-cyclonon-2,2-ylidene; 1,4-diaza-7-oxacyclonon-1,4-ylene; 1,4-diaza-7-oxa-cyclonon- 1,2-ylene; 1,4diaza-7-oxa-cyclonon-2,3-ylene; 1,4-diaza-7-oxa-cyclonon-6, 8-ylene; 1,4-diaza-7-oxa-cyclonon-2,2-ylidene; 1,4-dioxan-2,3-ylene; 1,4- dioxan-2,6-ylene; 1,4-dioxan-2,2-ylidene; tetrahydropyran-2,3-ylene; tetrahydropyran-2,6-ylene; tetrahydropyran-2,5-ylene; tetrahydropyran-2,2- ylidene; 1,4,7-trithia-cyclonon-2,3-ylene; 1,4,7-trithia-cyclonon-2,9- ylene; und 1,4,7-trithia-cyclonon-2,2-ylidene,
      heterocycloalkyl: ausgewählt aus der Gruppe enthaltend: pyrrolinyl; pyrrolidinyl; morpholinyl; piperidinyl; piperazinyl; hexamethylene imine; 1,4-piperazinyl; tetrahydrothiophenyl; tetrahydrofuranyl; 1,4,7- triazacyclononanyl; 1,4,8,11-tetraazacyclotetradecanyl; 1,4,7,10,13- pentaazacyclopentadecanyl; 1,4-diaza-7-thiacyclononanyl; 1,4-diaza-7-oxa- cyclononanyl; 1,4,7,10-tetraazacyclododecanyl; 1,4-dioxanyl; 1,4,7- trithiacyclononanyl; tetrahydropyranyl; und oxazolidinyl, wobei das Heterocycloalkyl mit der Verbindung über jedes Atom im Ring des ausgewählten Heterocycloalkyls verbunden sein kann.
      amine: die Gruppe -N(R)2 wobei jedes R unabhängig ausgewählt ist aus: Wasserstoff; C1-C6-alkyl; C1-C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
      halogen: ausgewählt aus der Gruppe enthaltend: F; Cl; Br und I,
      halogenalkyl: ausgewählt aus der Gruppe enthaltend mono, di, tri-, poly und perhalogenated lineare und verzweigte C1-C8-alkyl
      pseudohalogen: ausgewählt aus der Gruppe enthaltend -CN, -SCN, -OCN, N3, -CNO, -SeCN sulphonate: die Gruppe -S(O)2OR, wobei R ausgewählt ist aus: Wasserstoff; Cl- C6-alkyl; phenyl; C1-C6-alkyl-C6H5; Li; Na; K; Cs; Mg; und Ca,
      sulphate: die Gruppe -OS(O)2OR, wobei R ausgewählt ist aus: Wasserstoff; Cl- C6-alkyl; phenyl; C1-C6-alkyl-C6H5; Li; Na; K; Cs; Mg; und Ca,
      sulphone: die Gruppe -S(O)2R, wobei R ausgewählt ist aus: Wasserstoff; C1-C6- alkyl; phenyl; C1-C6-alkyl-C6H5 und amine (to give sulphonamide) ausgewählt aus der Gruppe: - NR'2, wobei jedes R' unabhängig ausgewählt ist aus: Wasserstoff; C1-C6-alkyl; C1C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
      carboxylat: die Gruppe -C(O)OR, wobei R ausgewählt ist aus: Wasserstoff; C1-C6-alkyl; phenyl; C1-C6-alkyl-C6H5; Li; Na; K; Cs; Mg; und Ca,
      carbonyl: die Gruppe -C(O)R, wobei R ausgewählt ist aus: Wasserstoff; C1-C6-alkyl; phenyl; C1-C6-alkyl-C6H5 und amine (to give amide) ausgewählt aus der Gruppe: -NR'2, wobei jedes R' unabhängig ausgewählt ist aus: Wasserstoff; Cl- C6-alkyl; C1-C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
      phosphonate: die Gruppe -P(O) (OR) 2, wobei jedes R unabhängig ausgewählt ist aus: Wasserstoff; C1-C6-alkyl; phenyl; C1-C6-alkyl-C6H5; Li; Na; K; Cs; Mg; und Ca,
      phosphate: die Gruppe -OP(O)(OR)2, wobei jedes R unabhängig ausgewählt ist aus: Wasserstoff; C1-C6-alkyl; phenyl; C1-C6-alkyl-C6H5; Li; Na; K; Cs; Mg; und Ca, phosphine: die Gruppe -P(R)2, wobei jedes R unabhängig ausgewählt ist aus: Wasserstoff; C1-C6-alkyl; phenyl; und C1-C6-alkyl-C6H5,
      phosphine oxid: die Gruppe -P (O) R2, wobei R unabhängig ausgewählt ist aus: Wasserstoff; C1-C6-alkyl; phenyl; und C1-C6-alkyl-C6H5; und amine (to give phosphonamidate) ausgewählt aus der Gruppe: -NR'2, wobei jedes R' unabhängig ausgewählt ist aus: Wasserstoff; C1-C6-alkyl; C1-C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet.
      polyether: ausgewählt aus der Gruppe enthaltend -(O-CH₂-CH(R))ₙ-OH und -(O-CH₂-CH(R))ₙ-H wobei R unabhängig ausgewählt ist aus: Wasserstoff, alkyl, aryl, halogen und n ist von 1 to 250
      silylalkyl: die Gruppe -SiR₃, wobei jedes R unabhängig voneinander ausgewählt ist aus: Wasserstoff; C1-C6- alkyl; phenyl; C1-C6-alkyl-C6H5 und amine (to give sulphonamide) ausgewählt aus der Gruppe: -NR'2, wobei jedes R' unabhängig ausgewählt ist aus: Wasserstoff; C1-C6-alkyl; C1C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet
      silylalkyloxy: die Gruppe -OSiR₃ , wobei jedes R unabhängig voneinander ausgewählt ist aus: Wasserstoff; C1-C6- alkyl; phenyl; C1-C6-alkyl-C6H5 und amine (to give sulphonamide) ausgewählt aus der Gruppe: -NR'2, wobei jedes R' unabhängig ausgewählt ist aus: Wasserstoff; C1-C6-alkyl; C1C6-alkyl-C6H5; und phenyl, wobei wenn beide R' C1-C6 alkyl sind, beide R' einen -NC3 bis NC5 heterocyclischen Ring bilden können, wobei die restliche Alkylkette einen Alkylsubstituenten am heterocyclischen Ring bildet

Soweit nicht anders erwähnt, sind die folgenden Gruppen mehr bevorzugte Gruppen innerhalb der allgemeinen Gruppendefinition:
alkyl: lineare und verzweigte C1-C6-alkyl,
langkettige Alkyle: lineare und verzweigte C5-C10 alkyl, vorzugsweise C6-C8 alkyle alkenyl: C3-C6-alkenyl,
cycloalkyl: C6-C8-cycloalkyl,
alkoxy: Cl-C4-alkoxy,
langkettig Alkoxy: lineare und verzweigte C5-C10 alkoxy, vorzugsweise lineare C6-C8 alkoxy
alkylene: ausgewählt aus der Gruppe enthaltend: methylene; 1,2-ethylene; 1,3-propylene; butan-2-ol-1,4-diyl; 1,4-butylene; cyclohexane-1,1-diyl; cyclohexan-1,2-diyl; cyclohexan-1,4-diyl; cyclopentane-1,1-diyl; und cyclopentan-1,2-diyl,
aryl: ausgewählt aus der Gruppe enthaltend: phenyl; biphenyl; naphthyl; anthracenyl; und phenanthrenyl,
arylene: ausgewählt aus der Gruppe enthaltend: 1,2-phenylene; 1,3- phenylene; 1,4-phenylene; 1,2-naphtalenylene; 1,4-naphtalenylene; 2,3- naphtalenylene und 1-hydroxy-2,6-phenylene,
heteroaryl: ausgewählt aus der Gruppe enthaltend:
   pyridinyl; pyrimidinyl; chinoninyl; pyrazolyl; triazolyl; isochinoninyl; imidazolyl; und oxazolidinyl, wobei das Heteroaryl mit der Verbindung über jedes Atom im Ring des ausgewählten Heteroaryls verbunden sein kann,
   heteroarylene: ausgewählt aus der Gruppe enthaltend: pyridin 2,3-diyl; pyridin-2,4-diyl; pyridin-2,6-diyl; pyridin-3,5-diyl; quinolin-2,3-diyl; quinolin-2,4-diyl; isoquinolin-1,3-diyl; isoquinolin-1,4-diyl; pyrazol-3,5-diyl; und imidazole-2,4-diyl,
   heterocycloalkyl: ausgewählt aus der Gruppe enthaltend:
      pyrrolidinyl; morpholinyl; piperidinyl; piperidinyl; 1,4 piperazinyl; tetrahydrofuranyl; 1,4,7-triazacyclononanyl; 1,4,8,11-tetraazacyclotetradecanyl; 1,4,7,10,13-pentaazacyclopentadecanyl; 1,4,7,10-tetraazacyclododecanyl; und piperazinyl, wobei das Heteroaryl mit der Verbindung über jedes Atom im Ring des ausgewählten Heteroaryls verbunden sein kann
   heterocycloalkylene: ausgewählt aus der Gruppe enthaltend:
      piperidin-2,6-ylene; piperidin-4,4-ylidene; 1,4-piperazin-1,4-ylene; 1,4-piperazin-2,3-ylene; 1,4-piperazin-2,6-ylene; tetrahydrothiophen-2,5-ylene; tetrahydrothiophen-3,4-ylene; tetrahydrofuran-2,5-ylene; tetrahydrofuran-3,4-ylene; pyrrolidin-2,5-ylene; pyrrolidin-2,2-ylidene; 1,4,7-triazacyclonon-1,4- ylene; 1,4,7-triazacyclonon-2,3-ylene; 1,4,7-triazacyclonon-2,2-ylidene; 1,4,8,11- tetraazacyclotetradec-1,4-ylene; 1,4,8,11-tetraazacyclotetradec-1,8-ylene; 1,4,8,11-tetraazacyclotetradec-2,3-ylene; 1,4,8,11-tetraazacyclotetradec-2,2-ylidene; 1,4,7,10-tetraazacyclododec-1,4-ylene; 1,4,7,10-tetraazacyclododec-1,7-ylene; 1,4,7,10-tetraazacyclododec-2,3-ylene; 1,4,7,10-tetraazacyclododec-2,2-ylidene; 1,4,7,10,13- pentaazacyclopentadec-1,4-ylene; 1,4,7,10,13-pentaazacyclopentadec-1,7-ylene; 1,4-diaza-7-thia-cyclonon-1,4 ylene; 1,4-diaza-7-thia-cyclonon-2,3-ylene; 1,4-diaza-7-thiein cyclonon-2,2-ylidene; 1,4-diaza-7-oxa-cyclonon-1,4-ylene; 1,4 diaza-7-oxa-cyclonon-2,3-ylene;1,4-diaza-7-oxa-cyclonon-2,2- ylidene; 1,4-dioxan-2,6-ylene; 1,4-dioxan-2,2-ylidene; tetrahydropyran-2,6-ylene; tetrahydropyran-2,5-ylene; und tetrahydropyran- 2,2-ylidene, ein -C1-C6-alkyl-heterocycloalkyl, wobei das Heterocycloalkyl ausgewählt aus der Gruppe enthaltend: piperidinyl; 1,4-piperazinyl; tetrahydrofuranyl; 1,4,7- triazacyclononanyl; 1,4,8,11-tetraazacyclotetradecanyl; 1,4,7,10,13-pentaazacyclopentadecanyl; 1,4,7,10-tetraazacyclododecanyl; und pyrrolidinyl, wobei das Heterocycloalkyl mit der Verbindung über jedes Atom im Ring des ausgewählten Heterocycloalkyls verbunden sein kann
   amine: die Gruppe -N (R) 2, wobei jedes R unabhängig ausgewählt ist aus: Wasserstoff; C1-C6-alkyl; und benzyl,
   halogen: ausgewählt aus der Gruppe enthaltend: F und Cl,
   sulphonate: die Gruppe -S(O)2OR, wobei R ausgewählt ist aus: Wasserstoff; Cl- C6-alkyl; Na; K; Mg; und Ca,
   sulphate: die Gruppe -OS(O)2OR, wobei R ausgewählt ist aus: Wasserstoff; Cl- C6-alkyl; Na; K; Mg; und Ca,
   sulphone: die Gruppe -S(O)2R, wobei R ausgewählt ist aus: Wasserstoff; C1-C6- alkyl; benzyl und amine ausgewählt aus der Gruppe: -NR'2, wobei jedes R' unabhängig ausgewählt ist aus: Wasserstoff; C1-C6-alkyl; und benzyl,
   carboxylat: die Gruppe -C(O)OR, wobei R ausgewählt ist aus Wasserstoff; Na; K; Mg; Ca; C1-C6-alkyl; und benzyl,
   carbonyl: die Gruppe: -C(O)R, wobei R ausgewählt ist aus: Wasserstoff; C1-C6-alkyl; benzyl und amine ausgewählt aus der Gruppe: -NR'2, wobei jedes R' unabhängig ausgewählt ist aus: Wasserstoff; C1-C6-alkyl; und benzyl,
   phosphonate: die Gruppe -P(O) (OR)2, wobei jedes R unabhängig ausgewählt ist aus: Wasserstoff; C1-C6-alkyl; benzyl; Na; K; Mg; und Ca,
   phosphate: die Gruppe -OP(O) (OR)2, wobei jedes R unabhängig ausgewählt ist aus: Wasserstoff; C1-C6-alkyl; benzyl; Na; K; Mg; und Ca,
   phosphine: die Gruppe -P(R)2, wobei jedes R unabhängig ausgewählt ist aus: Wasserstoff; C1-C6-alkyl; und benzyl,
   phosphine oxid: die Gruppe -P(O)R2, wobei R unabhängig ausgewählt ist aus: Wasserstoff; C1-C6-alkyl; benzyl und amine ausgewählt aus der Gruppe: -NR'2, wobei jedes R' unabhängig ausgewählt ist aus: Wasserstoff; C1-C6-alkyl; und benzyl.
   polyether: ausgewählt aus der Gruppe enthaltend-(O-CH₂-CH(R))ₙ-OH und -(O-CH₂-CH(R))ₙ-H wobei R unabhängig ausgewählt ist aus: Wasserstoff, methyl, halogen und n ist von 5 bis 50, bevorzugt 10 bis 25.
   M, Mₙ (n ist eine ganze Zahl) : Metalle, wobei zwei Metalle M unabhängig voneinander ausgewählt sind, wenn nicht anders angezeigt.

Gemäß einer bevorzugten Ausführungsform der Erfindung umfasst der Katalysator mindestens einen N-heterocyclischen Carbenliganden der folgenden Struktur: 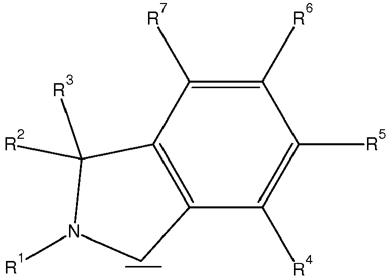 wobei
R¹ bis R³ unabhängig voneinander Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei können, wobei bei geeigneten Resten eine oder mehrere nicht-benachbarte CH₂-Gruppen unabhängig voneinander durch -O-, -S-, -NH-, -NR°-, -SiR°R°°-, -CO-, -COO-, -OCO-, -OCO-O-, -SO₂-, -S-CO-, -CO-S-, -CY¹=CY² oder -C=C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
R⁴ bis R⁷ unabhängig voneinander Wasserstoff, Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei können, wobei bei geeigneten Resten eine oder mehrere nicht-benachbarte CH₂-Gruppen unabhängig voneinander durch -O-, -S-, -NH-, -NR°-, -SiR°R°°-, -CO-, -COO-, -OCO-, -OCO-O-, -SO₂-, -S-CO-, -CO-S-, -CY¹=CY² oder -C=C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
und R¹ bis R⁷ so substituiert sein können, dass sich wahlweise zwischen R² und R³, R¹ und R²/R³ oder R²/R³ und R⁷ oder R⁶ und R⁷ oder R⁵ und R⁶ oder R⁴ und R⁵ ein Ring bildet.

Diese beiden vorigen N-heterocyclischen Carbenliganden sind besonders bevorzugt.

Gemäß einer bevorzugten Ausführungsform der Erfindung umfasst der Katalysator mindestens einen N-heterocyclischen Carbenliganden der folgenden Struktur: 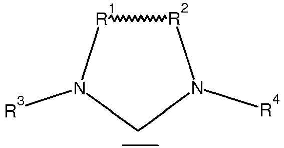 wobei
R¹ oder R² entweder ein substituierter oder unsubstituierter Kohlenstoff oder ein Stickstoff sein können (wobei aber R¹ und R² nicht beide Stickstoff sind), wobei die Substitutionen ausgewählt sind aus (unabhängig voneinander) Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei könen, wobei bei geeigneten Resten eine oder mehrere nicht-benachbarte CH₂-Gruppen unabhängig voneinander durch -O-, -S-, -NH-, - NR°-, -SiR°R°°-, -CO-, -COO-, -OCO-, -OCO-O-, -SO₂-, -S-CO-, -CO-S-, -CY¹=CY² oder - C=C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
R³ und R⁴ unabhängig voneinander Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei können, wobei bei geeigneten Resten eine oder mehrere nicht-benachbarte CH₂-Gruppen unabhängig voneinander durch -O-, -S-, -NH-, -NR°-, -SiR°R°°-, -CO-, -COO-, -OCO-, -OCO-O-, -SO₂-, -S-CO-, -CO-S-, -CY¹=CY² oder -C=C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
wobei die Bindung zwischen R¹ und R² eine Einfach- oder Doppelbindung sein kann
und R¹ und R³ einerseits und/oder R² und R⁴ anderseits so substituiert sein können, dass sich zwischen R¹ und R³ bzw. R² und R⁴ ein Ring bildet.

Gemäß einer bevorzugten Ausführungsform umfasst der Katalysator einen N-heterocyclischen Carbenliganden der folgenden Struktur: 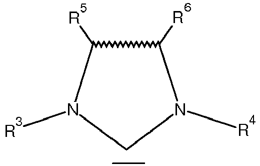
wobei R³ und R⁴ wie beim vorigen Katalysator definiert sind, R⁵ und R⁶ unabhängig voneinander Wasserstoff, Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei könen, wobei bei geeigneten Resten eine oder mehrere nicht-benachbarte CH₂-Gruppen unabhängig voneinander durch -O-, -S-, -NH-, -NR°-, -SiR°R°°-, -CO-, -COO-, -OCO-, -OCO-O-, -SO₂-, -S-CO-, -CO-S-, -CY¹=CY² oder -C=C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
und die Bindung zwischen den Ringkohlenstoffen eine Einfach- oder Doppelbindung sein kann.

Gemäß einer bevorzugten Ausführungsform der Erfindung umfasst der Katalysator mindestens einen N-heterocyclischen Carbenliganden der folgenden Struktur: 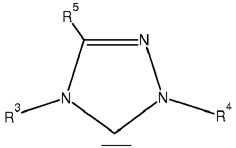 wobei R³, R⁴ und R⁵ wie beim vorigen Katalysator definiert sind.

Gemäß einer bevorzugten Ausführungsform der Erfindung umfasst der Katalysator mindestens einen N-heterocyclischen Carbenliganden der folgenden Struktur: 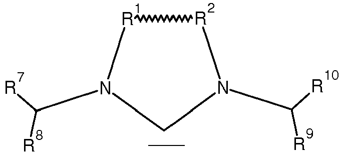 R¹ oder R² entweder ein substituierter oder unsubstituerter Kohlenstoff oder ein Stickstoff sein können (wobei aber R¹ und R² nicht beide Stickstoff sind) wobei die Bindung zwischen R¹ und R² eine Einfach- oder Doppelbindung sein kann wobei R⁷, R⁸, R⁹ und R¹⁰ unabhängig voneinander Wasserstoff, Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei könen, wobei bei geeigneten Resten eine oder mehrere nicht-benachbarte CH₂-Gruppen unabhängig voneinander durch -O-, -S-, -NH-, - NR°-, -SiR°R°°-, -CO-, -COO-, -OCO-, -OCO-O-, -SO₂-, -S-CO-, -CO-S-, -CY¹=CY² oder - C=C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)

Gemäß einer bevorzugten Ausführungsform der Erfindung umfasst der Katalysator mindestens einen N-heterocyclischen Carbenliganden der folgenden Struktur: 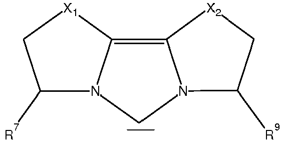 wobei R⁷ und R⁹ wie beim vorigen Katalysator definiert sind und X₁ und X₂ unabhängig voneinander O, NH oder CH₂ sein können.

Gemäß einer bevorzugten Ausführungsform der Erfindung umfasst der Katalysator am Rhodium noch mindestens einen weiteren Liganden, bevorzugt sind dabei labile Liganden.

Darunter werden Liganden verstanden, deren Bindung zum Rhodium so schwach sind, dass sie ggf. im Verlauf der Reaktion zumindest temporär nicht mehr an das Rhodium gebunden sind.

Bevorzugte derartige labile Liganden sind Dialkene, bevorzugt COD, oder Norbornadien, oder alternativ zu Dialkenen zwei Ethylene. Weiterhin mögliche Liganden sind Carbonylverbindungen, zwei Chloridverbindungen, Acetylacetonat, Hydridliganden, Halogenliganden, insbesondere Chlorid, Triflat, Acetonitril, schwach koordinierende Anionen wie Tetrafluoroborat, oder zwei Phosphanverbindungen.

Bevorzugt beträgt die Menge an Katalysator vor Reaktionsbeginn von ≥0,001 mol% bis ≤10 mol% (bezogen auf die aromatische Vorläufersubstanz bei Beginn der Reaktion), noch bevorzugt ≥0,05 mol% bis ≤5 mol%, ferner bevorzugt ≥0,1 mol% bis ≤3 mol% sowie am meisten bevorzugt ≥0,2 mol% bis ≤1 mol%. Es hat sich herausgestellt, dass auch bei diesen relativ kleinen Katalysatormengen trotzdem die Reaktion oftmals mit großer Ausbeute durchgeführt werden kann.

Bevorzugt wird das Verfahren in einem organischen Lösemittel durchgeführt, besonders bewährt in der Praxis haben sich (und sind somit bevorzugt): Hexan, Cyclohexan, Dichlormethan, Dichlorethan, Dioxan, Ethylacetat, Diethylether, THF, Aceton, Trifluorethanol oder Mischungen dieser Lösemittel. Besonders bevorzugt sind Hexan und Cyclohexan.

Bevorzugt wird das Verfahren bei einer Temperatur von ≥0°C durchgeführt. Die Obergrenze ist grundsätzlich nur durch die Siedetemperatur des verwendeten Lösemittels beschränkt. Noch bevorzugter sind Temperaturen von ≥10°C bis ≤50°C, am meisten bevorzugt Raumtemperatur, also ≥20°C bis ≤30°C

Für den Fall, dass gasförmiger Wasserstoff als Reduktionsmittel verwendet wird, ist der bevorzugte H₂-Druck (bei Beginn der Reaktion) ≥3 bar, besonders bevorzugt sind Bereiche von ≥10 bar bis ≤ 150 bar.

Die Reaktion wird bevorzugt für eine Reaktionszeit von ≥3h bis ≤2d durchgeführt, besonders bevorzugte Reaktionszeiten sind ≥5h bis ≤ 30h.

Bevorzugt wird die Reaktion in Gegenwart einer Lewis Säure durchgeführt, bevorzugt einer Lewis-Säure, welche zustätzlich wasserentziehende Eigenschaften besitzt. Es hat sich herausgestellt, dass so oftmals die Ausbeute noch weiter erhöht werden kann. Bevorzugte Additive sind ausgewählt aus der Gruppe enthaltend Molsieb, bevorzugt Molsieb 4 Å oder Molsieb 3 Å, Silicagel, Al₂O₃, Aluminiumtriisopropylat, TiO₂, Florisil® oder Mischungen daraus.

Bevorzugt beträgt die Menge an Lewissäure von ≥70 bis ≤ 600 mg, besonders bevorzugt ≥150 bis ≤ 450 mg pro 1 mmol Edukt.

Die vorgenannten sowie die beanspruchten und in den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Vorteile des Gegenstandes der Erfindung ergeben sich aus den Unteransprüchen sowie aus der nachfolgenden Beschreibung der zugehörigen Beispiele, in denen - beispielhaft - mehrere Ausführungsbeispiele des erfindungsgemäßen Verfahrens dargestellt sind.

### Allgemeine Arbeitsvorschrift I:

Ein ofengetrocknetes 9 mL Schraubdeckelgläschen befüllt mit Rührfisch und aktiviertem 4 Å Molsieb (100 mg) wurde unter Vakuum abkühlen gelassen und mit Katalysator (0,1 bis 1,5 mol%) und gg. festem Edukt (1.0 mmol) - wenn das verwendete Edukt fest ist - an der Luft befüllt. Die Atmosphäre im Gläschen wurde mit Argon ausgetauscht (3 x). Nach Zugabe von Hexan (2 mL) und anschließend gg. flüssigem Edukt (1.0 mmol) - wenn das verwendete Edukt flüssig ist - wurde das Schraubdeckelgläschen unter Argon in einen 150 mL Stahl-Autoklaven unter Argonatmosphäre transferiert, wobei während der Überführung der Autoklavendeckel geöffnet und der Autoklav mittels eines Argon-durchströmten Schlauches unter Schutzgas gehalten wurde. Die Atmosphäre im Autoklaven wurde mit Wasserstoffgas ausgetauscht (3 x), bevor 50 bar Wasserstoffdruck zugegeben und das Gemisch für 24 h bei 25 °C in einem Metallblock gerührt wurde (die angegebene Temperatur ist die des Metallblockes). Nach vorsichtigem Ablassen des Druckes wurde das Gemisch über Whatman Filter© filtriert, wobei mit Aceton nachgewaschen wurde (4x 1 mL). Das Lösungsmittel wurde bei 45 °C und 500 mbar am Rotationsverdampfer entfernt und das Rohprodukt säulenchromatographisch aufgereinigt (Kieselgel, Pentan/Diethylether oder Dichlormethan/Aceton abhängig von der Polarität), wobei das Lösungsmittel anschließend vorsichtig am Rotationsverdampfer entfernt wurde. Trocknung am Hochvakuum wurde angesichts der Flüchtigkeit der meisten Produkte nicht vorgenommen. Besonders flüchtige Produkte wurden destillativ gereinigt. Die angegebenen Diastereoselektivitäten beziehen sich auf die isolierten Produkte und wurden aus dem ¹H bzw. ¹⁹F NMR Spektrum bestimmt.

Anhand der allgemeinen Arbeitsvorschrift I wurden folgende fluorierte cyclische aliphatische Verbindungen hergestellt, wobei jeweils der folgende Katalysator ((2-(2,6-diisopropylphenyl)-3,3-dimethyl-2-azaspiro[4.5]decan-1-yliden)(cyclooctadienyl)rhodium(I) chlorid) verwendet wurde:

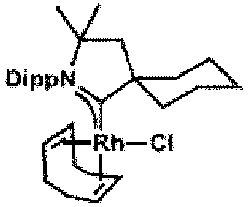

Die Ergebnisse sind in der folgenden Tabelle zusammengefasst

| Beispiel No. | Edukt | Produkt | Bedingungen und Ausbeute |
|---|---|---|---|
| 1 | 4-Fluorbenzoesäuremethylester | Methyl-*cis*-4-Fluorocyclohexancarboxylat | Katalysatorbeladung: 0,5 mol%. Farblose Flüssigkeit; 80% Ausbeute, 9:1 d.r. |
| | 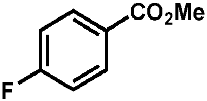 | 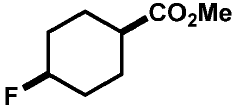 | |
| 2 | *tert-butyl(2-*fluorophenoxy)dime thylsilane | *tert*-butyl((*cis*-2-fluorocyclohexyl)oxy)dimethylsilan | Katalysatorbeladung: 0,1 mol%. Farblose Flüssigkeit; 96% Ausbeute, 16:1 d.r. |
| | 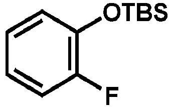 | 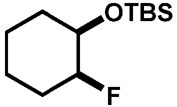 | |
| 3 | 2-(4-fluorophenyl)-4,4,5,5-tetramethyl-1,3,2-dioxaborolan | 2-(*cis*-4-fluorocyclohexyl)-4,4,5,5-tetramethyl-1,3,2-dioxaborolan | Katalysatorbeladung: 0,5 mol%. Farblose Flüssigkeit; 88% Ausbeute, 9:1 d.r. |
| | 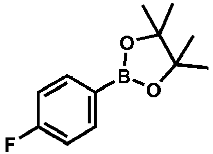 | | |
| | | 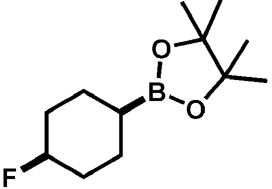 | |
| 4 | *tert-butyl* (4-fluorophenyl)carba mat | *tert*-butyl (*cis*-4-fluorocyclohexyl)carbamat | Katalysatorbeladung: 0,5 mol%. Weißer Feststoff; 81% Ausbeute, 6:1 d.r. |
| | 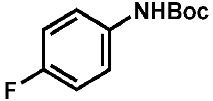 | 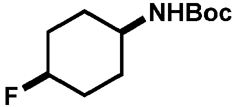 | |
| 5 | Fluorbenzol | Fluorcyclohexan | Katalysatorbeladung: 0,1 mol%. GC-Ausbeute. (Produkt ist bekannt und eine farblose Flüssigkeit) |
| | 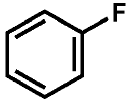 | 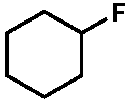 | |
| 6 | 1,2,3-Trifluorbenzol | All-*cis*-1,2,3-Trifluorcyclohexan | Katalysatorbeladung: 0,1 mol%. Weißer Feststoff; 81% Ausbeute. Das Produkt wurde über Kieselgel filtriert (Eluent: Diethylether) und das Lösungsmittel über eine Vigreux Kolonne abdestilliert. |
| | 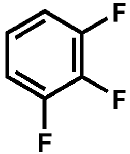 | 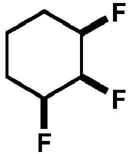 | |
| 7 | 1,3,5-Trifluorbenzol | All-cis-1,3,5-trifluorcyclohexan | Katalysatorbeladung: 0,1 mol%. Weißer Feststoff; 65% Ausbeute. Das Produkt wurde über Kieselgel filtriert (Eluent: Diethylether) und das Lösungsmittel über eine Vigreux Kolonne abdestilliert. |
| | 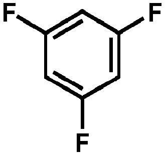 | 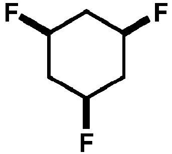 | |
| 8 | 1,2,4,5-Tetrafluorobenzol | All-*cis*-1,2,4,5-Tetrafluorcyclohexan | Katalysatorbeladung: 0,5 mol%. Weißer Feststoff; 60% Ausbeute. |
| | 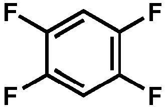 | 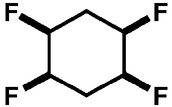 | |
| 9 | Pentafluorbenzol | All-*cis*-1,2,3,4,5-pentafluorcyclohexan | Katalysatorbeladung: 0,5 mol%. Weißer Feststoff; 42% Ausbeute. |
| | 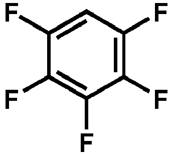 | 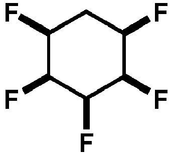 | |
| 10 | Hexafluorbenzol | All-*cis*-1,2,3,4,5,6-hexafluorcyclohexan | Katalysatorbeladung: 0.5 mol%. Weißer Feststoff; 22% Ausbeute. |
| | 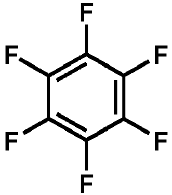 | 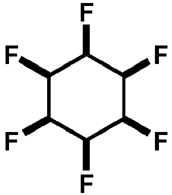 | |

### Allgemeine Arbeitsvorschrift II

Ein bei 135 °C ofengetrocknetes Reaktionsgefäß (abhängig von der Konzentration entweder ein 9 mL Schraubdeckelgläschen (2,0 mL Hexan, 0,5 M) oder ein 50 mL Glaszylinder (15 mL Hexan, 0.07 M)) befüllt mit Magnetrührfisch und aktiviertem 4 Å Molekularsieb oder Silica gel (150 mg oder, bei multifluorierten Aromaten 450 mg bei 135 °C ofengetrocknetes Kieselgel) wurde unter Vakuum abkühlen gelassen und mit Katalysator (0,10 bis 1,0 mol%) und ggf. festem Edukt (1,0 mmol) - wenn das verwendete Edukt fest ist - an der Luft befüllt. Die Atmosphäre im Gläschen wurde über ein Septum mit Argon ausgetauscht (3 x Vakuum/Argon Zyklus). Nach Zugabe von Hexan (2,0 mL oder 15 mL) und anschließend gg. flüssigem Edukt (1,0 mmol) - wenn das verwendete Edukt flüssig ist - wurde das Gefäß unter Argon in einen 150 mL Stahl- Autoklaven unter Argonatmosphäre transferiert, wobei während der Überführung der Autoklavendeckel geöffnet und der Autoklav mittels eines Argon-durchströmten Schlauches unter Schutzgas gehalten wurde. Die Atmosphäre im Autoklaven wurde mit Wasserstoffgas ausgetauscht (3 x 10 bar Wasserstoffdruck/Entlasten Zyklus), bevor 50 bar Wasserstoffdruck eingestellt und das Gemisch für 24 h bei 25 °C in einem Metallblock gerührt wurde (die angegebene Temperatur ist die des Metallblockes). Nach vorsichtigem Ablassen des Druckes wurde das Gemisch säulenchromatographisch aufgereinigt (Kieselgel, Pentan/Diethylether oder Dichlormethan/Aceton abhängig von der Polarität), wobei das Lösungsmittel anschließend vorsichtig am Rotationsverdampfer entfernt wurde. Trocknung am Hochvakuum wurde angesichts der Flüchtigkeit der meisten Produkte nicht vorgenommen. Besonders flüchtige Produkte wurden destillativ gereinigt. Die angegebenen Diastereoselektivitäten wurden per GC MS, GC-FID oder aus dem ¹⁹F NMR Spektrum direkt nach der Reaktion bestimmt. Die Notation >20:1 d.r. gibt an, dass nur ein einziges Diastereomer detektierbar war. Anhand der allgemeinen Arbeitsvorschrift wurden folgende fluorierte cyclische aliphatische Verbindungen hergestellt, wobei jeweils der folgende Katalysator ((2-(2,6-Diisopropylphenyl)-3,3-dimethyl-2-azaspiro[4.5]decan-1-yliden)(cyclooctadienyl)rhodium(I)chlorid) verwendet wurde:

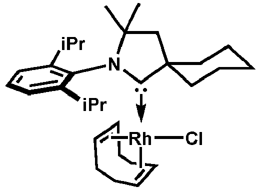

Die Ergebnisse sind in der folgenden Tabelle zusammengefasst:

| Beispiel No. | Edukt | Produkt | Bedingungen und Ausbeute |
|---|---|---|---|
| 1 | tert-Butyl(4-fluorphenoxy)dimeth ylsilan | *tert*-Butyl((*cis*-4-fluorcyclohexyl)oxy)di methylsilan | Farbloses Öl Katalysatorbeladung: 0,10 mol% Konzentration: 0,5 M 93% Ausbeute, 13:1 d.r. Die Reaktion wurde ebenfalls auf größerem Maßstab durchgeführt: 5,0 mmol Substrat Katalysatorbeladung: 0,10 mol% Konzentration: 0,5 M 400 mg Molekularsieb 98% Ausbeute, 11:1 d.r. |
| | 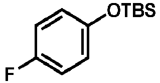 | 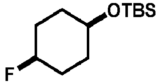 | |
| 2 | *tert-* Butyl (2-fluorphenoxy)dimeth ylsilan | *tert*-Butyl((*cis*-2-fluorcyclohexyl)oxy)di methylsilan | Farbloses Öl Katalysatorbeladung: 0,10 mol% Konzentration: 0,5 M 96% Ausbeute, 16:1 d.r. |
| | 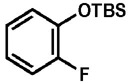 | 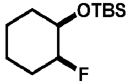 | |
| 3 | 2-(4-Fluorphenyl)-4,4,5,5-tetramethyl-1,3,2-dioxaborolan | 2-(*cis*-4-Fluorcyclohexyl)-4,4,5,5-tetramethyl-1,3,2-dioxaborolan | Farbloses Öl Katalysatorbeladung: 0,50 mol% Konzentration: 0,5 M 88% Ausbeute, 9:1 d.r. |
| | 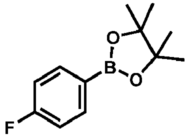 | 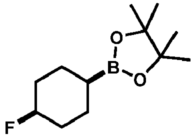 | |
| 4 | 2-(3-Fluorphenyl)-4,4,5,5-tetramethyl-1,3,2-dioxaborolan | *2-(cis-3-*Fluorcyclohexyl)-4,4,5,5-tetramethyl-1,3,2-dioxaborolan | Farbloses Öl Katalysatorbeladung: 0,50 mol% Konzentration: 0,5 M 82% Ausbeute, 8:1 d.r. |
| | 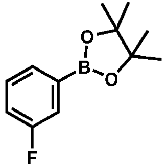 | 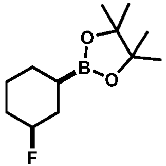 | |
| 5 | 2-(2-Fluorphenyl)-4,4,5,5-tetramethyl-1,3,2-dioxaborolan | *2-(cis-2-*Fluorcyclohexyl)-4,4,5,5-tetramethyl-1,3,2-dioxaborolan | Farbloses Öl Katalysatorbeladung: 0,50 mol% Konzentration: 0,5 M 61% Ausbeute, >20:1 d.r. Das Produkt zersetzt sich langsam bei Raumtemperatur. |
| | 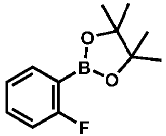 | 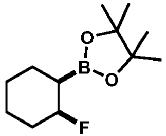 | |
| 6 | *tert*-Butyl (4-fluorphenyl)carbama t | *tert*-Butyl (*cis*-4-fluorcyclohexyl)carbam at | Weißer Feststoff Katalysatorbeladung: 0,50 mol% Konzentration: 0,5 M 81% Ausbeute, 6:1 d.r. Die Reaktion wurde ebenfalls auf größerem Maßstab durchgeführt: 10 mmol Substrat Katalysatorbeladung: 0,50 mol% Konzentration: 0,5 M in 7:1 Hexane/Dichlormethan 400 mg Molekularsieb 47% Ausbeute, 7:1 d.r. |
| | 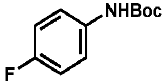 | 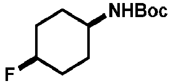 | |
| 7 | *tert-Butyl* (2-fluorphenyl)carbama t | *tert-Butyl (cis-2-*fluorcyclohexyl)carbam at | Weißer Feststoff Katalysatorbeladung: 0,50 mol% Konzentration: 0,5 M 63% Ausbeute, >20:1 d.r. |
| | 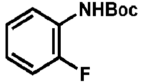 | 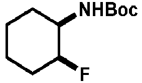 | |
| 8 | 4-Fluoranisol | *cis*-1-Fluor-4-methoxycyclohexan | Farbloses Öl Katalysatorbeladung: 0,50 mol% Konzentration: 0,5 M 80% Ausbeute, 7:1 d.r. Nach Filtration über Kieselgel wurde das Hexan als Azeotrop mit Aceton über eine Vigreux Kolonne abdestilliert. |
| | 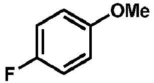 | 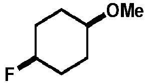 | |
| 9 | 4-Fluorbenzoesäuremethylester | Methyl-*cis*-4-fluorcyclohexancarboxy lat | Farbloses Öl Katalysatorbeladung: 0,50 mol% Konzentration: 0,5 M 80% Ausbeute, 9:1 d.r. |
| | 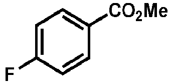 | 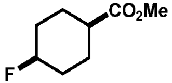 | |
| 10 | *tert-Butyl* (4-fluorbenzyl)carbamat | *tert*-Butyl ((*cis*-4-fluorcyclohexyl)methyl )carbamat | Farbloses Öl Katalysatorbeladung: 0,50 mol% Konzentration: 0,5 M 90% ¹⁹F NMR Ausbeute (mit Hexafluorbenzol als internem Standard), 7:1 d.r. Das Produkt wurde zusammen mit Spuren des Eduktes (ca. 7%) isoliert. |
| | 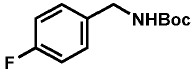 | 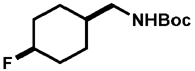 | |
| 11 | 2-(2,4-Difluorphenyl)-4,4,5,5-tetramethyl-1,3,2-dioxaborolan | 2-(All-*cis*-2,4-difluorcyclohexyl)-4,4,5,5-tetramethyl-1,3,2-dioxaborolan | Farbloses Öl Katalysatorbeladung: 1,0 mol% Konzentration: 0,07 M 71% Ausbeute, 9:1 d.r. |
| | 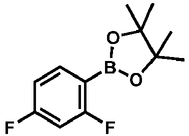 | 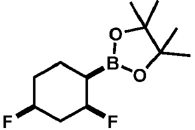 | Das Produkt zersetzt sich langsam bei Raumtemperatur. |
| 12 | *tert-*Butyldimethyl(3,4,5-trifluorphenoxy) silan | tert-Butyldimethyl((all-*cis*-3,4,5-trifluorcyclohexyl)oxy) silan | Weißer Feststoff Katalysatorbeladung: 1,0 mol% Konzentration: 0,07 M 68% Ausbeute, 7:1 d.r. |
| | 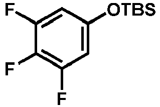 | 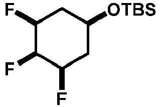 | |
| 13 | *tert-*Butyldimethyl(2,4,6-trifluorphenoxy) silan | tert-Butyldimethyl((all-*cis*-2,4,6-trifluorcyclohexyl)oxy) silan | Farbloses Öl Katalysatorbeladung: 1,0 mol% Konzentration: 0,07 M 97% Ausbeute, >20:1 d.r. |
| | 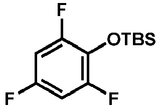 | 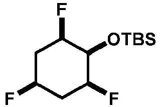 | |
| 14 | ((2-Fluor-1,4-phenylen)bis(oxy))bi s(*tert-*butyldimethylsilan) | ((All-*cis*-2-Fluorcyclohexan-1,4-diyl)bis(oxy))bis(*tert-*butyldimethylsilan) | Farbloses Öl Katalysatorbeladung: 1,0 mol% Konzentration: 0,07 M 38% Ausbeute, >20:1 d.r. Das Produkt wurde zusammen mit Spuren des defluorierten Produktes (*cis*-1,4-Bis((*tert-*butyldimethylsilyl)oxy)cyclohex an) isoliert. |
| | 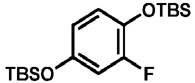 | 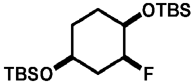 | |
| 15 | ((Perfluor-1,4-phenylen)bis(oxy))bi | ((All-cis-2,3,5,6-tetrafluorcyclohexan-1,4-diyl)bis(oxy))bis(*tert-*butyldimethylsilan) | Weißer Feststoff Katalysatorbeladung: 1,0 mol% |
| | s(*tert-*butyldimethylsilan) | | Konzentration: 0,07 M 21% Ausbeute, >20:1 d.r. |
| | 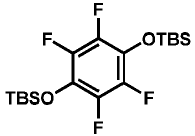 | 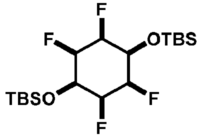 | |
| 16 | Methyl 4-fluor-2-hydroxybenzoat | Methyl 4-fluor-2-hydroxycyclohex-1-en-1-carboxylat | Gelbliches Öl Katalysatorbeladung: 1,0 mol% Konzentration: 0,07 M 42% Ausbeute Das angegebene Enol wurde zusammen mit Spuren (<5%) des vollständig hydrierten Alkohols isoliert. |
| | 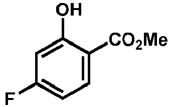 | 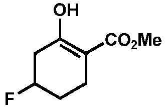 | |
| 17 | Fluorbenzol | Fluorcyclohexan | Katalysatorbeladung: 0,10 mol% Konzentration: 0,5 M 90% GC-FID Ausbeute (mit Mesitylen als internem Standard) Das Produkt ist bekannt und eine farblose flüchtige Flüssigkeit. |
| | 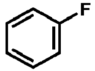 | 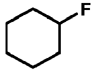 | |
| 18 | 1,2-Difluorbenzol | *cis-1,2-*Difluorcyclohexan | Katalysatorbeladung: 0,10 mol% Konzentration 0,5 M 96% ¹⁹F NMR Ausbeute (mit Hexafluorbenzol als internem Standard), 17:1 d.r. In einem weiteren Versuch |
| | 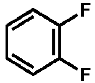 | 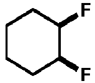 | |
| | | | wurde die Reaktion in Pentan durchgeführt. Nach beendeter Reaktion wurde das Gemisch über Kieselgel filtriert und das Lösungsmittel vorsichtig entfernt. Das leicht flüchtige Produkt wurde mit 72% Ausbeute und 17:1 d.r. gewonnen und war mit Spuren (ca. 1.0%) an Fluorcyclohexan verunreinigt. |
| 19 | 1,2,3-Trifluorbenzol | All-*cis*-1,2,3-trifluorcyclohexan | Farbloses Öl Katalysatorbeladung: 0,10 mol% Konzentration: 0,5 M 81% Ausbeute, 6:1 d.r. Das Reaktionsgemisch wurde über Kieselgel filtriert (Eluent: Diethylether) und das Lösungsmittel über eine Vigreux Kolonne abdestilliert. Die Reaktion wurde ebenfalls auf größerem Maßstab durchgeführt: 25 mmol Substrat Katalysatorbeladung: 0,10 mol% Konzentration: 1,0 M 400 mg Molekularsieb 86% Ausbeute, 7:1 d.r. Nach Abdestillieren des |
| | 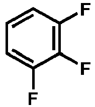 | 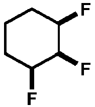 | |
| | | | Lösungsmittels wurde das Produkt über eine Destillation aufgereinigt. |
| 20 | 1,3,5-Trifluorbenzol | All-*cis*-1,3,5-trifluorcyclohexan | Weißer Feststoff Katalysatorbeladung: 0,10 mol% Konzentration: 0,5 M 65% Ausbeute (97% GC-FID Ausbeute mit Mesitylen als internem Standard), >20:1 d.r. Das Reaktionsgemisch wurde über Kieselgel filtriert (Eluent: Diethylether) und das Lösungsmittel über eine Vigreux Kolonne abdestilliert. |
| | 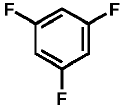 | 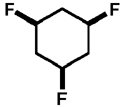 | |
| 21 | 1,2,4,5-Tetrafluorbenzol | All-*cis*-1,2,4,5-tetrafluorcyclohexan | Weißer Feststoff Katalysatorbeladung: 0,50 mol% Konzentration: 0,07 M 60% Ausbeute, >20:1 d.r. |
| | 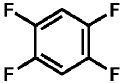 | 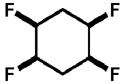 | |
| 22 | 1,2,3,4-Tetrafluorbenzol | All-*cis*-1,2,3,4-tetrafluorcyclohexan | Weißer Feststoff Katalysatorbeladung: 0,50 mol% Konzentration: 0,5 M 26% Ausbeute, >20:1 d.r. |
| | 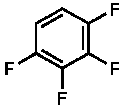 | 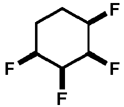 | |
| 23 | Pentafluorbenzol | All-*cis*-1,2,3,4,5-pentafluorcyclohexan | Weißer Feststoff Katalysatorbeladung: 1,0 mol% Konzentration: 0,5 M 42% Ausbeute, >20:1 d.r. Die Reaktion wurde ebenfalls mit 450 mg bei 135 °C |
| | 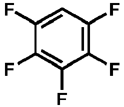 | 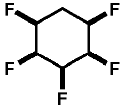 | |
| | | | ofengetrocknetem Kieselgel statt Molekularsieb durchgeführt. (89% Ausbeute, >20:1 d.r.) |
| 24 | Hexafluorbenzol | All-*cis*-1,2,3,4,5,6-hexafluorcyclohexan | Weißer Feststoff Katalysatorbeladung: 0,50 mol% Konzentration: 0,07 M 34% Ausbeute, >20:1 d.r. Die Reaktion wurde ebenfalls mit gepulvertem aktiviertem Molsieb durchgeführt (89% NMR Ausbeute). Die Reaktion wurde ebenfalls mit 450 mg bei 135 °C ofengetrocknetem Kieselgel statt Molekularsieb durchgeführt. (88% Ausbeute, >20:1 d.r.) Die Reaktion wurde ebenfalls auf einem 20 mmol Maßstab (0,50 mol% Katalysatorbeladung, 35 mL Hexan, 4,5 g Kieselgel, 62% Ausbeute) durchgeführt. |
| | 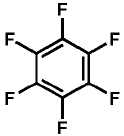 | 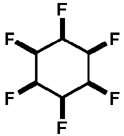 | |
| 25 | Perfluornaphthalin | All-*cis*-1,2,3,4,5,6,7,8-octafluor-1,2,3,4-tetrahydronaphthalin | Weißer Feststoff Katalysatorbeladung: 0,50 mol% Konzentration: 0,14 M 56% Ausbeute, >20:1 d.r. |
| | 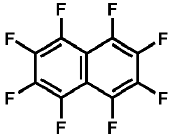 | | |
| | | 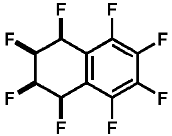 | |
| 26 | Perfluor-1,1'-biphenyl | 1,2,3,4,5-Pentafluor-6-(all-cis-2,3,4,5,6-pentafluorcyclohexyl)b enzol | Weißer Feststoff Katalysatorbeladung: 0,50 mol% Konzentration: 0,07 M 12% Ausbeute, >20:1 d.r. |
| | | | |
| | 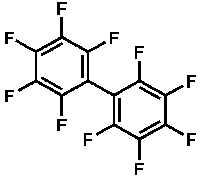 | 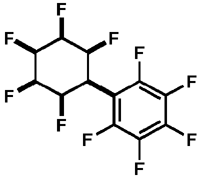 | |
| 27 | 4,4'-Difluor-1,1'-biphenyl | All-*cis*-4,4'-difluor-1,1'-bi(cyclohexan) | Weißer Feststoff Katalysatorbeladung: 0,50 mol% Konzentration: 0,5 M 96% Ausbeute, 15:1 d.r. Die Verbindung wurde als 7:1 Mischung zweier stabiler Konfomere erhalten. |
| | 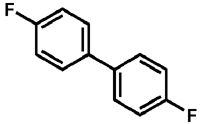 | 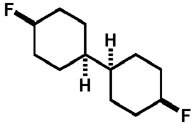 | |
| 28 | 1-Fluornaphthalin | All-*cis*-1-fluordec ahydronaphthal in | Farbloses Öl Katalysatorbeladung: 1,0 mol% Konzentration: 0,5 M 74% Ausbeute, 6:1 d.r. |
| | 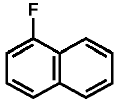 | | |
| | | 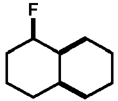 | |
| 29 | 2-Fluor-1,1'-biphenyl | *cis*-2-Fluor-1,1'-bis (cyclohexan) | Farbloses Öl Katalysatorbeladung: 0,50 mol% Konzentration: 0,5 M 91% Ausbeute, 20:1 d.r. |
| | 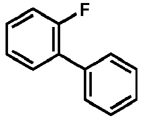 | 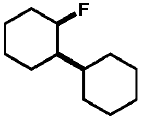 | |
| 30 | *tert*-Butyl 6-fluor-1*H*-indol-1-carboxylat | *tert*-Butyl all-*cis*-6-fluorctahydro-1*H*-indol-1-carboxylat | Farbloses Öl Katalysatorbeladung: 1,0 mol% Konzentration: 0,07 M 83% Ausbeute, 1,5:1 d.r. |
| | | | |
| | 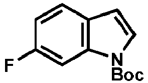 | 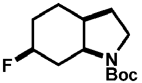 | |
| 31 | tert-Butyl 5-fluor-1*H*-indol-1-carboxylat | *tert*-Butyl all-*cis*-5-fluorctahydro-1*H*-indol-1-carboxylat | Farbloses Öl Katalysatorbeladung: 1,0 mol% Konzentration: 0,07 M |
| | 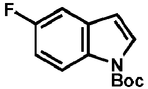 | 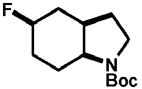 | 52% Ausbeute, 6:1 d.r. Bei 135 °C ofengetrocknetes Kieselgel (150 mg) wurde statt Molekularsieb verwendet. |
| 32 | 5-Fluorbenzofuran | *All-cis-5-*fluorctahydrobenzofura n | Farbloses Öl Katalysatorbeladung: 1,0 mol% Konzentration: 0,07 M 85% Ausbeute, 8:1 d.r. |
| | 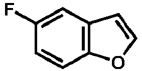 | | |
| | | 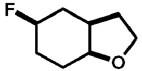 | |
| 33 | 3-Fluorpyridin-2(1*H*)-on | 3-Fluorpiperidin-2-on | Weißer Feststoff Katalysatorbeladung: 1,0 mol% Konzentration: 0,07 M 53% Ausbeute Bei 135 °C ofengetrocknetes Kieselgel (150 mg) wurde statt Molekularsieb verwendet. |
| | 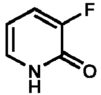 | 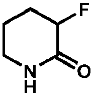 | |
| 34 | 3-Desoxy-3-fluorestron | 3-Desoxy-3-fluor-all-*cis*-hexahydroestron | Weißer Feststoff Katalysatorbeladung: 1,0 mol% Konzentration: 0,02 M 81% Ausbeute Die Reaktion wurde mit einer Ansatzgröße von 0,50 mmol des Substrats unter Verwendung von |
| | 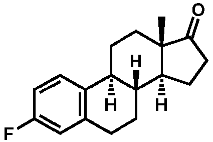 | 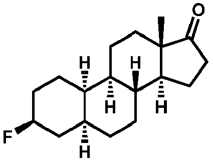 | |
| | | | bei 135 °C ofen getrocknetem Kieselgel (150 mg) durchgeführt. Das Verhältnis des Hauptdiastereomers zu allen anderen Diastereomeren ist 3:1. |

In einem weiteren Versuch wurde die Hydrierung von *tert*-Butyl(4-fluorphenoxy)dimethylsilan unter Verwendung des kationischen ((2-(2,6-Diisopropylphenyl)-3,3-dimethyl-2-azaspiro[4.5]decan-1-yliden)(cyclooctadienyl)rhodium(I)tetrafluorborat) Komplexes (0,10 mmol Substrat, Katalysatorbeladung: 5,0 mol%, 0,1 M in Hexan) in Abwesenheit von Molekularsieb oder vergleichbarer Additive gemäß der allgemeinen Versuchsvorschrift getestet. Es wurden 71% GC Ausbeute von *tert*-Butyl((*cis*-4-fluorcyclohexyl)oxy)dimethylsilan (17:1 d.r.) beobachtet.

Die Strukturformel des kationischen Komplexes ist wie folgt:

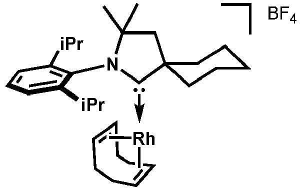

In einem weiteren Versuch wurde die Hydrierung von tert-Butyl(4-fluorphenoxy)dimethylsilan unter Verwendung des kationischen 1-(2,6-Diisopropylphenyl)-2,2,4,4-tetramethyl-3,4-dihydro-2H-pyrrol-1-yliden(cyclooctadienyl)rhodium(I)chlorid Komplexes (0,10 mmol Substrat, Katalysatorbeladung: 0,1 mol%, 0,05 M in Hexan) in Gegenwart von 4 Ä Molekularsieb (50 mg) gemäß der allgemeinen Versuchsvorschrift getestet. Es wurden 73% GC Ausbeute von *tert*-Butyl((*cis*-4-fluorcyclohexyl)oxy)-dimethylsilan (13:1 d.r.) beobachtet.

Die Strukturformel des verwendeten Komplexes ist wie folgt:

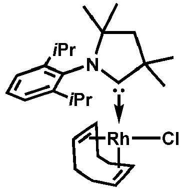

Ein weiterer Versuch wurde mit 1-(4-Fluorphenyl)-2-phenylethan-1-on als Edukt (0,10 mmol) sowie Trifluorethanol als Lösemittel (0,5 M) und 5,0 mol% (*R*)-(2-(3,3-dimethylbutan-2-yl)-3,3-diphenylisoindolin- 1-yliden)(cyclooctadienyl)rhodium(I)chlorid als Katalysator durchgeführt, die Strukturformel ist die folgende:

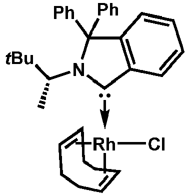

(Es wurde ein Gemisch der beiden Katalysatordiastereomere eingesetzt). Man erhielt 24% ¹⁹F NMR-Ausbeute an 2-Cyclohexyl-1-(4-fluorcyclohexyl)ethan-1-ol.

### Allgemeine Arbeitsvorschrift III:

Ein bei 135 °C ofengetrocknetes 9 mL Schraubdeckelgläschen befüllt mit Magnetrührfisch und aktiviertem 4 Å Molekularsieb oder Silica gel (250 mg) wurde unter Vakuum abkühlen gelassen und mit Katalysator (2,0 bis 3,0 mol%) und nicht flüchtigem Edukt (0,5 mmol) - wenn das verwendete Edukt nicht flüchtig ist - an der Luft befüllt. Die Atmosphäre im Gläschen wurde über ein Septum mit Argon ausgetauscht (3 x Vakuum/Argon Zyklus). Nach Zugabe des Lösungsmittels (Dichlormethan oder Mischungen aus Hexan und Dichlormethan, 2,0 mL) und anschließend gg. flüchtigem Edukt (0,5 mmol) - wenn das verwendete Edukt flüchtig ist - wurde das Gefäß unter Argon in einen 150 mL Stahl- Autoklaven unter Argonatmosphäre transferiert, wobei während der Überführung der Autoklavendeckel geöffnet und der Autoklav mittels eines Argon-durchströmten Schlauches unter Schutzgas gehalten wurde. Die Atmosphäre im Autoklaven wurde mit Wasserstoffgas ausgetauscht (3 x 10 bar Wasserstoffdruck/Entlasten Zyklus), bevor 50 bar Wasserstoffdruck eingestellt und das Gemisch für 24 h bei 25 °C in einem Metallblock gerührt wurde (die angegebene Temperatur ist die des Metallblockes). Nach vorsichtigem Ablassen des Druckes wurde das Gemisch säulenchromatographisch aufgereinigt (Kieselgel, Pentan/Ethylacetat abhängig von der Polarität), wobei das Lösungsmittel anschließend vorsichtig am Rotationsverdampfer entfernt wurde. Trocknung am Hochvakuum wurde angesichts der Flüchtigkeit der meisten Produkte nicht vorgenommen. Besonders flüchtige Produkte wurden destillativ gereinigt. Die angegebenen Diastereoselektivitäten wurden per GC MS direkt nach der Reaktion bestimmt.

Anhand der allgemeinen Arbeitsvorschrift wurden folgende fluorierte cyclische aliphatische Verbindungen hergestellt, wobei jeweils der folgende Katalysator ((2-(2,6-Diisopropylphenyl)-3,3-dimethyl-2-azaspiro[4.5]decan-1-yliden)(cyclo-octadienyl)rhodium(I)chlorid) verwendet wurde:

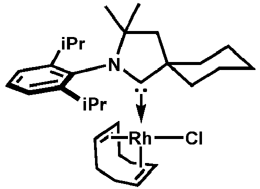

Die Ergebnisse sind in der folgenden Tabelle zusammengefasst:

| Beispiel No. | Edukt | Produkt | Bedingungen und Ausbeute |
|---|---|---|---|
| 1 | Triethoxy(3-fluorphenyl)silan | triethoxy(*cis*-3-fluorcyclohexyl)silan | Farbloses Öl Katalysatorbeladung: 3,00 mol% 72% Ausbeute, 8:1 d.r. |
| | 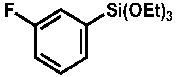 | 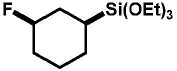 | Lösungsmittel: CH₂Cl₂/Hexan = 4:1 |
| 2 | (4-Fluorphenyl)-triethylsilan | (*cis*-4-Fluorocyclohexyl)trieth ylsilane | Farbloses Öl Katalysatorbeladung: 2,00 mol% Lösungsmittel: Hexan. 66% Ausbeute, 6:1 d.r. |
| | 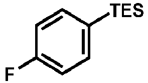 | | |
| | | 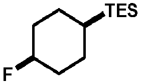 | |

Die einzelnen Kombinationen der Bestandteile und der Merkmale von den bereits erwähnten Ausführungen sind exemplarisch; der Austausch und die Substitution dieser Lehren mit anderen Lehren, die in dieser Druckschrift enthalten sind mit den zitierten Druckschriften werden ebenfalls ausdrücklich erwogen. Der Fachmann erkennt, dass Variationen, Modifikationen und andere Ausführungen, die hier beschrieben werden, ebenfalls auftreten können ohne von dem Erfindungsgedanken und dem Umfang der Erfindung abzuweichen. Entsprechend ist die obengenannte Beschreibung beispielhaft und nicht als beschränkend anzusehen. Das in den Ansprüchen verwendete Wort "umfassen" schließt nicht andere Bestandteile oder Schritte aus. Der unbestimmte Artikel "ein" schließt nicht die Bedeutung eines Plurals aus. Die bloße Tatsache, dass bestimmte Maße in gegenseitig verschiedenen Ansprüchen rezitiert werden, verdeutlicht nicht, dass eine Kombination von diesen Maßen nicht zum Vorteil benutzt werde kann. Der Umfang der Erfindung ist in den folgenden Ansprüchen definiert und den dazugehörigen Äquivalenten.

## Patentansprüche

1. Verfahren zur Herstellung fluorierter cyclischer aliphatischer Verbindungen umfassend den Schritt der Hydrierung einer aromatischen fluorhaltigen Vorläufersubstanz mit einem Katalysator, umfassend mindestens ein Rhodiumatom sowie mindestens einen N-Heterocyclischen Carbenliganden, in Gegenwart eines Reduktionsmittels.

2. Verfahren nach Anspruch 1, wobei der N-heterocyclische Carbenligand eine Verbindung ausgewählt aus der Gruppe enthaltend Pyrrolidinylidene, Pyrrolidene, Imidazolylidene, Imidazolidinylidene, Piperidinylene, Hexahydropyrimidinylene und Triazolylidene umfasst

3. Verfahren nach Anspruch 1 oder 2, wobei der N-heterocyclische Carbenligand eine Pyrrolidinyliden-Verbindung umfasst, bei der sich in α-Stellung sowohl zum Stickstoff wie zum Carben kein Wasserstoff befindet.

4. Verfahren nach einem der Ansprüche 1 bis 3, wobei der N-heterocyclische Carbenligand eine der folgenden Verbindungen umfasst: 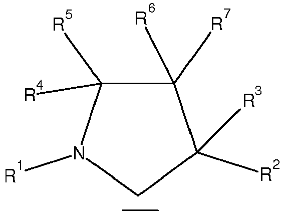 wobei
R¹ bis R⁵ unabhängig voneinander Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei können, wobei bei geeigneten Resten eine oder mehrere nicht-benachbarte CH₂-Gruppen unabhängig voneinander durch -O-, -S-, -NH-, -NR°-, - SiR°R°°-, -CO-, -COO-, -OCO-, -OCO-O-, -SO₂-, -S-CO-, -CO-S-, - CY¹=CY² oder -C≡C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
R⁶ und R⁷ unabhängig voneinander Wasserstoff, Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei können, wobei bei geeigneten Resten eine oder mehrere nicht-benachbarte CH₂-Gruppen unabhängig voneinander durch -O-, -S-, -NH-, -NR°-, - SiR°R°°-, -CO-, -COO-, -OCO-, -OCO-O-, -SO₂-, -S-CO-, -CO-S-, - CY¹=CY² oder -C≡C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
und R¹ bis R⁷ so substituiert sein können, dass sich wahlweise zwischen R² und R³, R⁴ und R³, R⁶ und R⁷, R¹ und R⁴/R⁵, R⁴/R⁵ und R⁶/R⁷ oder R²/R³ und R⁶/R⁷ ein Ring bildet; 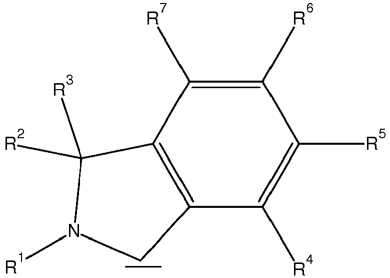
wobei
R¹ bis R³ unabhängig voneinander Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei können, wobei bei geeigneten Resten eine oder mehrere nicht-benachbarte CH₂-Gruppen unabhängig voneinander durch -O-, -S-, -NH-, -NR°-, - SiR°R°°-, -CO-, -COO-, -OCO-, -OCO-O-, -SO₂-, -S-CO-, -CO-S-, - CY¹=CY² oder -C≡C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
R⁴ bis R⁷ unabhängig voneinander Wasserstoff, Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei können, wobei bei geeigneten Resten eine oder mehrere nicht-benachbarte CH₂-Gruppen unabhängig voneinander durch -O-, -S-, -NH-, -NR°-, - SiR°R°°-, -CO-, -COO-, -OCO-, -OCO-O-, -SO₂-, -S-CO-, -CO-S-, - CY¹=CY² oder -C≡C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
und R¹ bis R⁷ so substituiert sein können, dass sich wahlweise zwischen R² und R³, R¹ und R²/R³ oder R²/R³ und R⁷ oder R⁶ und R⁷ oder R⁵ und R⁶ oder R⁴ und R⁵ ein Ring bildet; 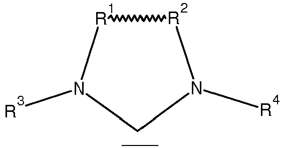
wobei
R¹ oder R² entweder ein substituierter oder unsubstituierter Kohlenstoff oder ein Stickstoff sein können (wobei aber R¹ und R² nicht beide Stickstoff sind), wobei die Substitutionen ausgewählt sind aus (unabhängig voneinander) Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl,
Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei könen, wobei bei geeigneten Resten eine oder mehrere nicht-benachbarte CH₂-Gruppen unabhängig voneinander durch -O-, - S-, -NH-, -NR°-, -SiR°R°°-, -CO-, -COO-, -OCO-, -OCO-O-, -SO₂-, -S-CO-, - CO-S-, -CY¹=CY² oder -C≡C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
R³ und R⁴ unabhängig voneinander Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei können, wobei bei geeigneten Resten eine oder mehrere nicht-benachbarte CH₂-Gruppen unabhängig voneinander durch -O-, -S-, -NH-, -NR°-, - SiR°R°°-, -CO-, -COO-, -OCO-, -OCO-O-, -SO₂-, -S-CO-, -CO-S-, - CY¹=CY² oder -C≡C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
wobei die Bindung zwischen R¹ und R² eine Einfach- oder Doppelbindung sein kann
und R¹ und R³ einerseits und/oder R² und R⁴ anderseits so substituiert sein können, dass sich zwischen R¹ und R³ bzw. R² und R⁴ ein Ring bildet. 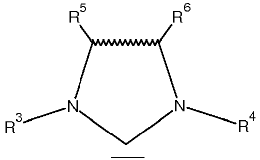
wobei R³ und R⁴ wie beim vorigen Katalysator definiert sind, R⁵ und R⁶ unabhängig voneinander Wasserstoff, Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei könen, wobei bei geeigneten Resten eine oder mehrere nicht-benachbarte CH₂-Gruppen unabhängig voneinander durch -O-, -S-, -NH-, -NR°-, -SiR°R°°-, - CO-, -COO-, -OCO-, -OCO-O-, -SO₂-, -S-CO-, -CO-S-, -CY¹=CY² oder - C=C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.)
und die Bindung zwischen den Ringkohlenstoffen eine Einfach- oder Doppelbindung sein kann; 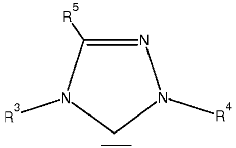
wobei R³, R⁴ und R⁵ wie beim vorigen Katalysator definiert sind; 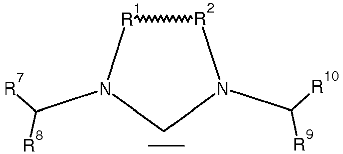
wobei R¹ oder R² entweder ein substituierter oder unsubstituierter Kohlenstoff oder ein Stickstoff sein können (wobei aber R¹ und R² nicht beide Stickstoff sind),
wobei die Bindung zwischen R¹ und R² eine Einfach- oder Doppelbindung sein kann,
wobei R⁷, R⁸, R⁹ und R¹⁰ unabhängig voneinander Wasserstoff, Alkyl, langkettiges Alkyl, Alkoxy, langkettiges Alkoxy, Cycloalkyl, Halogenalkyl, Aryl, Halogenaryl, Heteroaryl, Heterocycloalkylene, Heterocycloalkyl, Halogenheteroaryl, Alkenyl, Halogenalkenyl, Alkinyl, Halogenalkinyl, Ketoaryl, Halogenketoaryl, Ketoheteroaryl, Ketoalkyl, Halogenketoalkyl, Silylalkyl, Silylalkyloxy sei könen, wobei bei geeigneten Resten eine oder mehrere nicht-benachbarte CH₂-Gruppen unabhängig voneinander durch -O-, - S-, -NH-, -NR°-, -SiR°R°°-, -CO-, -COO-, -OCO-, -OCO-O-, -SO₂-, -S-CO-, - CO-S-, -CY¹=CY² oder -C≡C- ersetzt sein können und zwar derart, dass O und/oder S Atome nicht direkt miteinander verbunden sind, ebenfalls optional mit Aryl- oder Heteroaryl bevorzugt enthaltend 1 bis 30 C Atome ersetzt sind (endständige CH₃-Gruppen werden wie CH₂-Gruppen im Sinne von CH₂-H verstanden.);
sowie 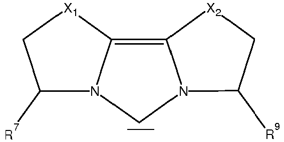 wobei R⁷ und R⁹ wie beim vorigen Katalysator definiert sind und X₁ und X₂ unabhängig voneinander O, NH oder CH₂ sein können.

5. Verfahren nach einem der Ansprüche 1 bis 4, wobei die Menge an Katalysator vor Reaktionsbeginn von ≥0,05 mol% bis ≤5 mol% (bezogen auf die aromatische Vorläufersubstanz bei Beginn der Reaktion) beträgt.

6. Verfahren nach einem der Ansprüche 1 bis 5, wobei das Verfahren in einem organischen Lösemittel durchgeführt wird.

7. Verfahren nach einem der Ansprüche 1 bis 6, wobei das Verfahren bei einer Temperatur von ≥0°C durchgeführt wird.

8. Verfahren nach einem der Ansprüche 1 bis 7, wobei das Verfahren unter Verwendung von gasförmigem Wasserstoff als Reduktionsmittel durchgeführt wird und der H₂-Druck (bei Beginn der Reaktion) ≥10 bar beträgt.

9. Verfahren nach einem der Ansprüche 1 bis 8, wobei die Reaktionszeit ≥4h bis ≤2d beträgt.

## Claims

1. Method for producing fluorinated cyclic aliphatic compounds comprising the step of hydrogenating an aromatic fluorine-containing precursor substance with a catalyst comprising at least one rhodium atom and at least one N-heterocyclic carbene ligand, in the presence of a reductant.

2. Method according to claim 1, wherein the N-heterocyclic carbene ligand is a compound selected from the group including pyrrolidinylidenes, pyrrolidenes, imidazolylidenes, imidazolidinylidenes, piperidinylenes, hexahydropyrimidinylene and triazolylidenes.

3. Method according to claim 1 or 2, wherein the N-heterocyclic carbene ligand comprises a pyrrolidinylidene compound in which there is no hydrogen in the α-position both to nitrogen and carbene.

4. Method according to any one of claims 1 to 3, wherein the N-heterocyclic carbene ligand comprises any of the following compounds: 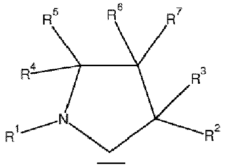 wherein:
R¹ to R⁵ independently of one another can be alkyl, long-chain alkyl, alkoxy, long-chain alkoxy, cycloalkyl, haloalkyl, aryl, haloaryl, heteroaryl, heterocycloalkylene, heterocycloalkyl, haloheteroaryl, alkenyl, haloalkenyl, alkynyl, haloalkynyl, ketoaryl, haloketoaryl, ketoheteroaryl, ketoalkyl, haloketoalkyl, silylalkyl, silylalkyloxy, wherein with suitable residues one or more non-adjacent CH₂ groups can be substituted independently of one another with -O-, -S-, -NH-, -NR°-, SiR°R°°-, -CO-, -COO-, -OCO-, -OCO-O-, -SO₂-, -S-CO-, -CO-S-, -CY¹=CY² or -C=C- in such a way that O and/or S atoms are not directly bound to one another, moreover, optionally are substituted with aryl or heteroaryl, preferably including 1 to 30 C atoms (terminal CH₃ groups are understood as CH₂ groups in the sense of CH₂-H),
R⁶ and R⁷ independently of one another can be hydrogen, alkyl, long-chain alkyl, alkoxy, long-chain alkoxy, cycloalkyl, haloalkyl, aryl, haloaryl, heteroaryl, heterocycloalkylene, heterocycloalkyl, haloheteroaryl, Alkenyl, haloalkenyl, alkynyl, haloalkynyl, ketoaryl, haloketoaryl, ketoheteroaryl, ketoalkyl, haloketoalkyl, silylalkyl, silylalkyloxy, wherein with suitable residues one or more non-adjacent CH₂ groups can be substituted independently of one another with -O-, -S-, -NH-, -NR°-, SiR°R°°-, -CO-, -COO-, -OCO-, - OCO-O-, -SO₂-, -S-CO-, -CO-S-, -CY¹=CY² or -C=C- in such a way that O and/or S atoms are not directly bound to one another, moreover, optionally are substituted with aryl or heteroaryl, preferably including 1 to 30 C atoms (terminal CH₃ groups are understood as CH₂ groups in the sense of CH₂-H),
and R¹ to R⁷ can be substituted such that optionally between R² and R³, R⁴ and R³, R⁶ and R⁷, R¹ and R⁴/R⁵, R⁴/R⁵ and R⁶/R⁷ or R²/R³ and R⁶/R⁷ a ring is formed; 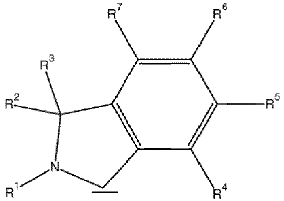
wherein R¹ to R³ independently of one another can be alkyl, long-chain alkyl, alkoxy, long-chain alkoxy, cycloalkyl, haloalkyl, aryl, haloaryl, heteroaryl, heterocycloalkylene, heterocycloalkyl, haloheteroaryl, alkenyl, haloalkenyl, alkynyl, haloalkynyl, ketoaryl, haloketoaryl, ketoheteroaryl, ketoalkyl, haloketoalkyl, silylalkyl, silylalkyloxy, wherein with suitable residues one or more non-adjacent CH₂ groups can be substituted independently of one another with -O-, -S-, -NH-, -NR°-, SiR°R°°-, -CO-, -COO-, -OCO-, - OCO-O-, -SO₂-, -S-CO-, -CO-S-, -CY¹=CY² or -C≡C- in such a way that O and/or S atoms are not directly bound to one another, moreover, optionally are substituted with aryl or heteroaryl, preferably including 1 to 30 C atoms (terminal CH₃ groups are understood as CH₂ groups in the sense of CH₂-H),
R⁴ to R⁷ independently of one another can be hydrogen, alkyl, long-chain alkyl, alkoxy, long-chain alkoxy, cycloalkyl, haloalkyl, aryl, haloaryl, heteroaryl, heterocycloalkylene, heterocycloalkyl, haloheteroaryl, alkenyl, haloalkenyl, alkynyl, haloalkynyl, ketoaryl, haloketoaryl, ketoheteroaryl, ketoalkyl, haloketoalkyl, silylalkyl, silylalkyloxy, wherein with suitable residues one or more non-adjacent CH₂ groups can be substituted independently of one another with -O-, -S-, -NH-, -NR°-, SiR°R°°-, -CO-, -COO-, -OCO-, - OCO-O-, -SO₂-, -S-CO-, -CO-S-, -CY¹=CY² or -C=C- in such a way that O and/or S atoms are not directly bound to one another, moreover, optionally are substituted with aryl or heteroaryl, preferably including 1 to 30 C atoms (terminal CH₃ groups are understood as CH₂ groups in the sense of CH₂-H),
and R¹ to R⁷ can be substituted such that optionally between R² and R³, R¹ and R²/R³ or R²/R³ and R⁷ or R⁶ and R⁷ or R⁵ and R⁶ or R⁴ and R⁵ a ring is formed; 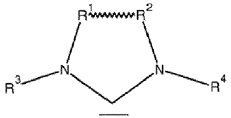
wherein:
R¹ or R² can be either a substituted or unsubstituted carbon or nitrogen (however, wherein R¹ and R² are not both nitrogen), wherein the substitutions are selected from (independently of one another) alkyl, long-chain alkyl, alkoxy, long-chain alkoxy, cycloalkyl, haloalkyl, aryl, haloaryl, heteroaryl, heterocycloalkylene, heterocycloalkyl, haloheteroaryl, alkenyl, haloalkenyl, alkynyl, haloalkynyl, ketoaryl, haloketoaryl, ketoheteroaryl, ketoalkyl, haloketoalkyl, silylalkyl, silylalkyloxy, wherein with suitable residues one or more non-adjacent CH₂ groups can be substituted independently of one another with -O-, -S-, -NH-, -NR°-, SiR°R°°-, -CO-, -COO-, -OCO-, - OCO-O-, -SO₂-, -S-CO-, -CO-S-, -CY¹=CY² or -C≡C- in such a way that O and/or S atoms are not directly bound to one another, moreover, optionally are substituted with aryl or heteroaryl, preferably including 1 to 30 C atoms (terminal CH₃ groups are understood as CH₂ groups in the sense of CH₂-H),
R³ and R⁴ independently of one another can be alkyl, long-chain alkyl, alkoxy, long-chain alkoxy, cycloalkyl, haloalkyl, aryl, haloaryl, heteroaryl, heterocycloalkylene, heterocycloalkyl, haloheteroaryl, alkenyl, haloalkenyl, alkynyl, haloalkynyl, ketoaryl, haloketoaryl, ketoheteroaryl, ketoalkyl, haloketoalkyl, silylalkyl, silylalkyloxy, wherein with suitable residues one or more non-adjacent CH₂ groups can be substituted independently of one another with -O-, -S-, -NH-, -NR°-, SiR°R°°-, -CO-, -COO-, -OCO-, -OCO-O-, -SO₂-, -S-CO-, -CO-S-, -CY¹=CY² or -C=C- in such a way that O and/or S atoms are not directly bound to one another, moreover, optionally are substituted with aryl or heteroaryl, preferably including 1 to 30 C atoms (terminal CH₃ groups are understood as CH₂ groups in the sense of CH₂-H),
wherein the bond between R¹ and R² can be a single or a double bond, and
R¹ and R³ on the one hand and/or R² and R⁴ on the other hand can be substituted in such a way that a ring is formed between R¹ and R³ or R² and R⁴; 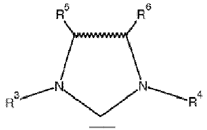
wherein R³ and R⁴ are defined as in the previous catalyst; R⁵ and R⁶ independently of one another can be hydrogen, alkyl, long-chain alkyl, alkoxy, long-chain alkoxy, cycloalkyl, haloalkyl, aryl, haloaryl, heteroaryl, heterocycloalkylene, heterocycloalkyl, haloheteroaryl, alkenyl, haloalkenyl, alkynyl, haloalkynyl, ketoaryl, haloketoaryl, ketoheteroaryl, ketoalkyl, haloketoalkyl, silylalkyl, silylalkyloxy, wherein with suitable residues one or more non-adjacent CH₂ groups can be substituted independently of one another with -O-, -S-, -NH-, -NR°-, SiR°R°°-, -CO-, -COO-, -OCO-, -OCO-O-, -SO₂-, -S-CO-, -CO-S-, -CY¹=CY² or -C=C- in such a way that O and/or S atoms are not directly bound to one another, moreover, optionally are substituted with aryl or heteroaryl, preferably including 1 to 30 C atoms (terminal CH₃ groups are understood as CH₂ groups in the sense of CH₂-H),
and the bond between the ring carbons can be a single or double bond; 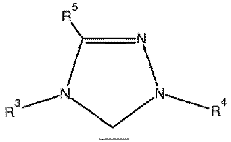
wherein R³, R⁴ and R⁵ are defined as in the previous catalyst; 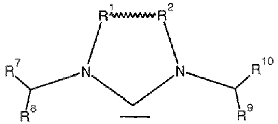
wherein R¹ or R² can be either a substituted or an unsubstituted carbon or nitrogen (however, wherein R¹ and R² are not both nitrogen),
wherein the bond between R¹ and R² can be a single or a double bond,
wherein R⁷, R⁸, R⁹ and R¹⁰ independently of one another can be hydrogen, alkyl, long-chain alkyl, alkoxy, long-chain alkoxy, cycloalkyl, haloalkyl, aryl, haloaryl, heteroaryl, heterocycloalkylene, heterocycloalkyl, haloheteroaryl, alkenyl, haloalkenyl, alkynyl, haloalkynyl, ketoaryl, haloketoaryl, ketoheteroaryl, ketoalkyl, haloketoalkyl, silylalkyl, silylalkyloxy, wherein with suitable residues one or more non-adjacent CH₂ groups can be substituted independently of one another with -O-, -S-, -NH-, -NR°-, SiR^{∘}R^{∘∘}-, -CO-, -COO-, -OCO-, -OCO-O-, -SO₂-, -S-CO-, -CO-S-, -CY¹=CY² or -C≡C-in such a way that O and/or S atoms are not directly bound to one another, moreover, optionally are substituted with aryl or heteroaryl, preferably including 1 to 30 C atoms (terminal CH₃ groups are understood as CH₂ groups in the sense of CH₂-H),
and 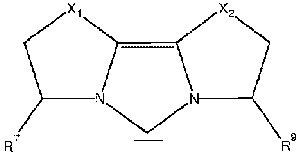 wherein R⁷ and R⁹ are defined as in the previous catalyst and X₁ and X₂ independently of one another can be O, NH or CH₂.

5. Method according to any one of claims 1 to 4, wherein the amount of catalyst before the start of the reaction is from ≥ 0.05 mol% to ≤ 5 mol% (based on the aromatic precursor substance at the start of the reaction).

6. Method according to any one of claims 1 to 5, wherein the method is carried out in an organic solvent.

7. Method according to any one of claims 1 to 6, wherein the method is carried out at a temperature of ≥ 0°C.

8. Method according to any one of claims 1 to 7, wherein the method is carried out by use of gaseous hydrogen as reductant and the H₂ pressure (at the start of the reaction) is ≥ 10 bar.

9. Method according to any one of claims 1 to 8, wherein the reaction time is ≥ 4h to ≤ 2d.

## Revendications

1. Procédé de fabrication de composés aliphatiques cycliques fluorés, comprenant l'étape d'hydrogénation d'une substance précurseur aromatique contenant du fluor avec un catalyseur comprenant au moins un atome de rhodium ainsi qu'au moins un ligand carbène N-hétérocyclique en présence d'un agent réducteur.

2. Procédé selon la revendication 1, dans lequel le ligand carbène N-hétérocyclique est un composé choisi dans le groupe comprenant des pyrrolidinylidènes, des pyrrolidènes, des imidazolylidènes, des imidazolidinylidènes, des pipéridynylènes, des hexahydropyrimidynylènes et des triazolylidènes

3. Procédé selon la revendication 1 ou la revendication 2, dans lequel le ligand carbène N-hétérocyclique comprend un composé pyrrolidinylidène dans lequel aucun hydrogène ne se trouve en position α ni de l'azote, ni du carbène.

4. Procédé selon l'une quelconques des revendications 1 à 3, dans lequel le ligand carbène N-hétérocyclique comprend un des composés suivants : 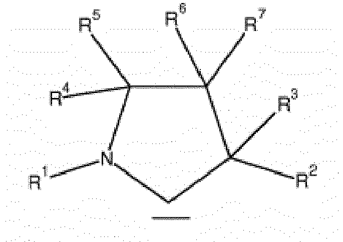 dans lequel
R¹ à R⁵ peuvent être, indépendamment l'un de l'autre, alkyle, alkyle à longue chaîne, alkoxy, alkoxy à longue chaîne, cycloalkyle, haloalkyle, aryle, haloaryle, hétéroaryle, hétérocycloalkylène, hétérocycloalkyle, halohétéroaryle, alcényle, haloalcényle, alcynyle, haloalcynyle, cétoaryle, halocétoaryle, cétohétéroaryle, cétoalkyle, halocétoalkyle, silylalkyle, silylalcoxy, dans lequel, dans le cas de radicaux appropriés, un ou plusieurs groupes CH₂ non adjacents peuvent être remplacés indépendamment les uns des autres par -O-, -S-, -NH-, -NR^{∘} -, - SiR^{∘}R^{∘∘}-, -CO-, -COO-, - OCO-, -OCO-O-, -SO₂-, -S-CO-, -CO-S-, - CY¹=CY² ou -C≡C-, et ce de telle sorte que les atomes O et/ou S ne sont pas directement liés les uns aux autres, et optionnellement sont également remplacés par un aryle ou un hétéroaryle comprenant de préférence 1 à 30 atomes de carbone (les groupes terminaux CH₃ sont entendus comme des groupes CH₂ au sens de CH₂-H.)
R⁶ et R⁷ peuvent être, indépendamment l'un de l'autre, hydrogène, alkyle, alkyle à longue chaîne, alcoxy, alcoxy à longue chaîne, cycloalkyle, haloalkyle, aryle, haloaryle, hétéroaryle, hétérocycloalkylène, hétérocycloalkyle, halohétéroaryle, alcényle, haloalcényle, alcynyle, haloalcynyle, cétoaryl, halocétoaryle, cétohétéroaryle, cétoalkyle, halocétoalkyle, silylalkyle, silylacoxy, dans lequel, dans le cas de radicaux appropriés, un ou plusieurs groupes CH₂ non adjacents peuvent être remplacés indépendamment les uns des autres par -O-, -S-, -NH-, -NR^{∘}-, - SiR^{∘}R^{∘∘}-, -CO-, -COO-, -OCO-, -OCO-O-, -SO₂-, -S-CO-, -CO-S-, - CY¹=CY² ou -C≡C-, et ce de telle sorte que les atomes O et/ou S ne sont pas directement liés les uns aux autres, et optionnellement sont également remplacés par un aryle ou un hétéroaryle comprenant de préférence 1 à 30 atomes de carbone (les groupes terminaux CH₃ sont entendus comme des groupes CH₂ au sens de CH₂-H.)
et R¹ à R⁷ peuvent être remplacés de sorte qu'un cycle se forme au choix entre R² et R³, R⁴ et R³, R⁶ et R⁷, R¹ et R⁴/R⁵, R⁴/R⁵ et R⁶/R⁷ ou R²/R³ et R⁶/R⁷ ; 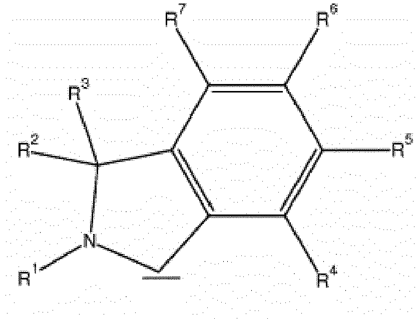
dans lequel
R¹ à R³ peuvent être, indépendamment l'un de l'autre, alkyle, alkyle à longue chaîne, alcoxy, alcoxy à longue chaîne, cycloalkyle, haloalkyle, aryle, haloaryle, hétéroaryle, hétérocycloalkylène, hétérocycloalkyle, halohétéroaryle, alcényle, haloalcényle, alcynyle, haloalcynyle, cétoaryl, halocétoaryle, cétohétéroaryle, cétoalkyle, halocétoalkyle, silylalkyle, silylacoxy, dans lequel, dans le cas de radicaux appropriés, un ou plusieurs groupes CH₂ non adjacents peuvent être remplacés indépendamment les uns des autres par -O-, -S-, -NH-, -NR^{∘}-, - SiR^{∘}R^{∘∘}-, -CO-, -COO-, -OCO-, -OCO-O-, -SO₂-, -S-CO-, -CO-S-, - CY¹=CY² ou -C≡C-, et ce de telle sorte que les atomes O et/ou S ne sont pas directement liés les uns aux autres, et optionnellement sont également remplacés par un aryle ou un hétéroaryle comprenant de préférence 1 à 30 atomes de carbone (les groupes terminaux CH₃ sont entendus comme des groupes CH₂ au sens de CH₂-H.)
R⁴ à R⁷ peuvent être, indépendamment l'un de l'autre, hydrogène, alkyle, alkyle à longue chaîne, alcoxy, alcoxy à longue chaîne, cycloalkyle, haloalkyle, aryle, haloaryle, hétéroaryle, hétérocycloalkylène, hétérocycloalkyle, halohétéroaryle, alcényle, haloalcényle, alcynyle, haloalcynyle, cétoaryl, halocétoaryle, cétohétéroaryle, cétoalkyle, halocétoalkyle, silylalkyle, silylalcoxy, dans lequel, dans le cas de radicaux appropriés, un ou plusieurs groupes CH₂ non adjacents peuvent être remplacés indépendamment les uns des autres par -O-, -S-, -NH-, -NR^{∘}-, - SiR^{∘}R^{∘∘}-, -CO-, -COO-, -OCO-, -OCO-O-, -SO₂-, -S-CO-, -CO-S-, - CY¹=CY² ou -C≡C-, et ce de telle sorte que les atomes O et/ou S ne sont pas directement liés les uns aux autres, et optionnellement sont également remplacés par un aryle ou un hétéroaryle comprenant de préférence 1 à 30 atomes de carbone (les groupes terminaux CH₃ sont entendus comme des groupes CH₂ au sens de CH₂-H.)
et R¹ à R⁷ peuvent être remplacés de sorte qu'un cycle se forme au choix entre R² et R³, R¹ et R²/R³ ou R²/R³ et R⁷ ou R⁶ et R⁷ ou R⁵ et R⁶ ou R⁴ et R⁵ ; 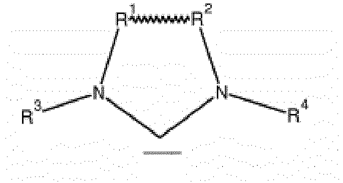
dans lequel
R¹ ou R² peuvent être soit un carbone substitué ou non substitué soit un azote substitué ou non substitué (dans lequel toutefois R¹ et R² ne sont pas tous deux azote), dans lequel les substitutions sont choisies (indépendamment l'une de l'autre) parmi alkyle, alkyle à longue chaîne, alcoxy, alcoxy à longue chaîne, cycloalkyle , haloalkyle, aryle, haloaryle, hétéroaryle, hétérocycloalkylènes, hétérocycloalkyle, halohétéroaryle, alcényle, haloalcényle, alcynyle, haloalcynyle, cétoaryle, halocétoaryle, cétohétéroaryle, cétoalkyle, halocétoalkyle, silylalkyle, silylalcoxy, dans lequel, dans le cas de radicaux appropriés, un ou plusieurs groupes CH₂ non adjacents peuvent être remplacés indépendamment les uns des autres par -O-, -S-, -NH-, -NR^{∘}-, - SiR^{∘}R^{∘∘}-, -CO-, -COO-, -OCO-, -OCO-O-, -SO₂-, -S-CO-, -CO-S-, - CY¹=CY² ou -C≡C-, et ce de telle sorte que les atomes O et/ou S ne sont pas directement liés les uns aux autres, et optionnellement sont également remplacés par un aryle ou un hétéroaryle comprenant de préférence 1 à 30 atomes de carbone (les groupes terminaux CH₃ sont entendus comme des groupes CH₂ au sens de CH₂-H.)
R³ et R⁴ peuvent être, indépendamment l'un de l'autre, alkyle, alkyle à longue chaîne, alcoxy, alcoxy à longue chaîne, cycloalkyle, haloalkyle, aryle, haloaryle, hétéroaryle, hétérocycloalkylène, hétérocycloalkyle, halohétéroaryle, alcényle, haloalcényle, alcynyle, haloalcynyle, cétoaryl, halocétoaryle, cétohétéroaryle, cétoalkyle, halocétoalkyle, silylalkyle, silylalcoxy, dans lequel, dans le cas de radicaux appropriés, un ou plusieurs groupes CH₂ non adjacents peuvent être remplacés indépendamment les uns des autres par -O-, -S-, -NH-, -NR^{∘}-, - SiR^{∘}R^{∘∘}-, -CO-, -COO-, -OCO-, -OCO-O-, -SO₂-, -S-CO-, -CO-S-, - CY¹=CY² ou -C≡C-, et ce de telle sorte que les atomes O et/ou S ne sont pas directement liés les uns aux autres, et optionnellement sont également remplacés par un aryle ou un hétéroaryle comprenant de préférence 1 à 30 atomes de carbone (les groupes terminaux CH₃ sont entendus comme des groupes CH₂ au sens de CH₂-H.)
dans lequel la liaison entre R¹ et R² peut être une liaison simple ou double et R¹ et R³ d'une part et/ou R² et R⁴ d'autre part peuvent être substitués de telle sorte qu'un cycle se forme entre R¹ et R³ ou R² et R⁴. 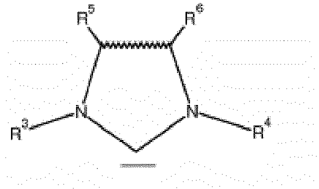
dans lequel R³ et R⁴ sont définis comme dans le cas du précèdent catalyseur, R⁵ et R⁶ peuvent être, indépendamment l'un de l'autre, hydrogène, alkyle, alkyle à longue chaîne, alcoxy, alcoxy à longue chaîne, cycloalkyle, haloalkyle, aryle, haloaryle, hétéroaryle, hétérocycloalkylène, hétérocycloalkyle, halohétéroaryle, alcényle, haloalcényle, alcynyle, haloalcynyle, cétoaryl, halocétoaryle, cétohétéroaryle, cétoalkyle, halocétoalkyle, silylalkyle, silylalcoxy, dans lequel, dans le cas de radicaux appropriés, un ou plusieurs groupes CH₂ non adjacents peuvent être remplacés indépendamment les uns des autres par -O-, -S-, -NH-, -NR°-, - SiR^{∘}R^{∘∘}-, -CO-, - COO-, -OCO-, -OCO-O-, -SO₂-, -S-CO-, -CO-S-, - CY¹=CY² ou -C≡C-, et ce de telle sorte que les atomes O et/ou S ne sont pas directement liés les uns aux autres, et optionnellement sont également remplacés par un aryle ou un hétéroaryle comprenant de préférence 1 à 30 atomes de carbone (les groupes terminaux CH₃ sont entendus comme des groupes CH₂ au sens de CH₂-H.)
et la liaison entre les carbones cycliques peut être une liaison simple ou double ; 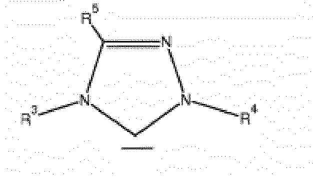
dans lequel R³, R⁴ et R⁵ sont définis comme dans le cas du précèdent catalyseur ; 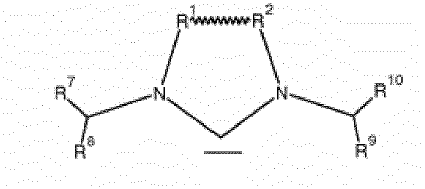
dans lequel R¹ ou R² peuvent être soit un carbone substitué ou non substitué soit un azote substitué ou non substitué (dans lequel toutefois R¹ et R² ne sont pas tous deux azote),
dans lequel la liaison entre R¹ et R² peut être une liaison simple ou double,
dans lequel R⁷, R⁸, R⁹ et R¹⁰ peuvent être, indépendamment l'un de l'autre, hydrogène, alkyle, alkyle à longue chaîne, alcoxy, alcoxy à longue chaîne, cycloalkyle, haloalkyle, aryle, haloaryle, hétéroaryle, hétérocycloalkylène, hétérocycloalkyle, halohétéroaryle, alcényle, haloalcényle, alcynyle, haloalcynyle, cétoaryl, halocétoaryle, cétohétéroaryle, cétoalkyle, halocétoalkyle, silylalkyle, silylalcoxy, dans lequel, dans le cas de radicaux appropriés, un ou plusieurs groupes CH₂ non adjacents peuvent être remplacés indépendamment les uns des autres par -O-, -S-, -NH-, -NR°-, - SiR^{∘}R^{∘∘}-, -CO-, -COO-, -OCO-, -OCO-O-, -SO₂-, -S-CO-, -CO-S-, - CY¹=CY² ou -C≡C-, et ce de telle sorte que les atomes O et/ou S ne sont pas directement liés les uns aux autres, et optionnellement sont également remplacés par un aryle ou un hétéroaryle comprenant de préférence 1 à 30 atomes de carbone (les groupes terminaux CH₃ sont entendus comme des groupes CH₂ au sens de CH₂-H.) ;
ainsi que 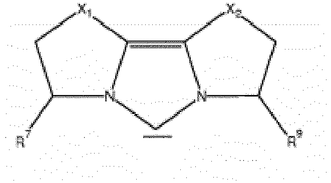 dans lequel R⁷ et R⁸ sont définis comme dans le cas du précèdent catalyseur et X₁ et X₂ peuvent être indépendamment l'un de l'autre O, NH ou CH₂.

5. Procédé selon l'une quelconque des revendications 1 à 4, dans lequel la quantité de catalyseur avant le début de la réaction s'élève de d≥ 0,05 mol% à ≤5 mol% (par rapport à la substance précurseur aromatique au début de la réaction).

6. Procédé selon l'une quelconque des revendications 1 à 5, dans lequel le procédé est mis en œuvre dans un solvant organique.

7. Procédé selon l'une quelconque des revendications 1 à 6, dans lequel le procédé est mis en œuvre à une température ≥ 0° C.

8. Procédé selon l'une quelconque des revendications 1 à 7, dans lequel le procédé est mis en œuvre en utilisant de l'hydrogène gazeux comme réducteur et la pression de H₂ (au début de la réaction) est ≥ 10 bars.

9. Procédé selon l'une quelconque des revendications 1 à 8, dans lequel le temps de réaction s'élève de ≥ 4h à ≤ 2j.
